# EUROPEAN PATENT SPECIFICATION

(11) **EP 1 032 571 B1**
(45) Date of publication and mention of the grant of the patent: **12.06.2002**
(21) Application number: 98947726.0
(22) Date of filing: 26.10.1998
(51) Int. Cl.: C07D 405/12, A61K 31/445, C07D 307/87, C07D 311/76

(54) **PIPERIDINYLAMINOMETHYL TRIFLUOROMETHYL CYCLIC ETHER COMPOUNDS AS SUBSTANCE P ANTAGONISTS**
CYCLISCHE PIPERIDINYLAMINOMETHYL-TRIFLUOROMETHYL-ETHERDERIVATE ALS SUBSTANZ P-ANTAGONISTEN
COMPOSES ETHER CYCLIQUES DU TYPE PIPERIDINYLAMINOMETHYLTRIFLUOROMETHYLE EM TANT QU'ANTAGONISTES DE LA SUBSTANCE P

(30) Priority: 19.11.1997 WO PCT/IB97/01466
(43) Date of publication of application: 06.09.2000
(73) Proprietor: PFIZER INC., New York, N.Y. 10017 (US)
(72) Inventor: SATAKE, Kunio, Chita-gun Aichi-ken 470-2393 (JP)
(74) Representative: Wood, David John
(86) International application number: IB9801704
(87) International publication number: WO9925714

(56) References cited:
- WO-A-95/06645
- WO-A-96/30367
- WO-A-97/03066
- WO-A-97/08144
- US-A- 5 604 247
- REINECKE M G ET AL: "Fluoroalkyl thieno- and benzo-fused.gamma.-lactones" ACTA CHEM. SCAND. (ACHSE7,0904213X);93; VOL.47 (3); PP.318-22, XP002093825 Texas Christian Univ.;Dep. Chem.; Fort Worth; 76129; TX; USA (US)
- CHEMICAL ABSTRACTS, vol. 094, no. 7, 16 February 1981 Columbus, Ohio, US; abstract no. 045498, HOSOKAWA K ET AL: "Synthesis of (trifluoromethyl)phthalic acids" XP002093826 & NIPPON KAGAKU KAISHI (NKAKB8,03694577);80; (8); PP.1304-6, Gov. Ind. Res. Inst.;Nagoya; 462; Japan

## Description

### Technical Field

This invention relates to novel piperidinylaminomethyl trifluoromethyl cyclic ether compounds and their pharmaceutically acceptable salts, pharmaceutical compositions containing such compounds, and the use of such compounds as substance P antagonists.

### Background Art

Substance P is a naturally occurring undecapeptide belonging to the tachykinin family of peptides, the latter being so-named because of their prompt stimulatory action on smooth muscle tissue. More specifically, substance P is a pharmaceutically active neuropeptide that is produced in mammals (having originally been isolated from gut) and possesses a characteristic amino acid sequence that is illustrated by D. F. Veber *et al.* in US Pat. 4680283. The wide involvement of substance P and other tachykinins in the pathophysiology of numerous diseases has been amply demonstrated in the art. For instance, substance P has recently been shown to be involved in the transmission of pain or migraine, as well as in central nervous system disorders such as anxiety and schizophrenia, in respiratory and inflammatory diseases such as asthma and rheumatoid arthritis, respectively, and in gastrointestinal disorders and diseases of the GI tract, like ulcerative colitis irritable bowel syndrome, Crohn's disease; etc. It is also reported that tachykinin antagonists are useful for the treatment of cardiovascular diseases, allergic conditions, immunoregulation, vasodilation, bronchospasm, reflex or neuronal control of the viscera, senile dementia of the Alzheimer type, emesis, sunburn and *Helicobacter pylori* infection.

International Patent Publication No. WO 97/08144 discloses a wide variety of substituted piperidine compounds, including piperidine compounds having a substituent comprising a fused ring moiety including an oxygen atom, as substance P antagonists. International Patent Publication No WO 96/30367 also discloses piperidine derivatives useful as substance P antagonists WO-A-95/06645 discloses benzofuran derivatives useful as tachykinin antagonists.

Substance P antagonists having improved activity and fewer side effects are desired.

### Brief Description of the Invention

The present invention provides piperidinylaminomethyl trifluoromethyl cyclic ether compounds of the following chemical formula (I): 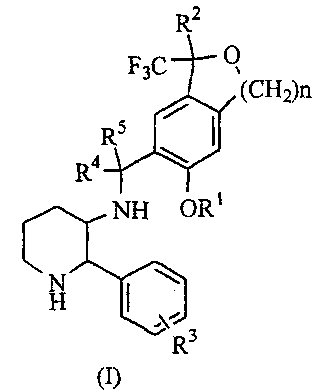 and their pharmaceutically acceptable salts, wherein
R¹ is C₁-C₆ alkyl;
R² is hydrogen, C₁-C₆ alkyl, halo C₁-C₆ alkyl or phenyl;
R³ is hydrogen or halo;
R⁴ and R⁵ are independently hydrogen, C₁-C₆ alkyl or halo C₁-C₆ alkyl; and
n is one, two or three.

These compounds are useful as substance P antagonists, and thus useful for treating a disorder or condition selected from cardiovascular diseases, allergic disorders, angiogenesis, gastrointestinal disorders, central nervous system disorders, inflammatory diseases, emesis, urinary incontinence, pain, migraine, severe anxiety disorders, stress disorders, anxiety, major depressive disorders, major depressive disorders with anxiety, depression, sunburn, sexual dysfunction, bipolar disorders, substance use disorders, schizophrenic disorders, movement disorders, cognitive disorders, and diseases, disorders and adverse conditions caused by *Helicobacter pylori,* or the like, in a mammal, especially a human. These compounds are especially useful as anti-inflammatory or anti-emetic agents, or agents for treating CNS disorders. Such CNS disorders include major depressive disorder, depression, major depressive disorder with anxiety, dysthemia, manic depression (bipolar or cyclothymic disorder), anxiety disorder, obsessive-compulsive disorder (OCD), panic disorder, phobias posttraumatic stress syndrome, neuralgia and cognitive disorders such as dementia and amnestic disorder. These compounds are also useful for Tourette's Syndrome, akinetic-rigid syndrome, movement disorders associated with Parkinson's disease, tardive dyskinesia and other dyskinesias. These compounds are particularly useful in the treatment of emesis, including acute, delayed or anticipatory emesis such as emesis or nausea induced by chemotherapy, radiation, surgery, pregnancy, motion, vestibular disorders, toxins, migraine, and variations in intercranical pressure. Most specifically, these compounds are of use in the treatment of emesis induced by antineoplastic agents, including those used in cancer therapy, and emesis induced by other pharmacological agents such as rolipram or morphine. These compounds are also useful as substance P antagonists with lower susceptibility to metabolism in a mammalian subject, especially a human. These compounds are also useful for chronic and acute pain including hyper-analgesic pain, neuropathic pain, post-operative pain and pain associated with nerve damage.

The present invention also relates to a pharmaceutical composition for treating a disorder or condition for which antagonist activity toward substance P is needed, in a mammal, which comprises an amount of the compound of Formula (I), or a pharmaceutically acceptable salt thereof, that is effective in treating such disorder or condition, and a pharmaceutically acceptable carrier.

The invention also relates to the use of a compound of Formula (I), or a pharmaceutically acceptable salt thereof, for the preparation of a medicament for treating a disorder or condition for which antagonist activity toward substance P.

The invention also relates to a pharmaceutical composition for treating a disorder or condition selected from cardiovascular diseases, allergic disorders, angiogenesis, gastrointestinal disorders, central nervous system disorders, inflammatory diseases, emesis, urinary incontinence, pain, migraine; severe anxiety disorders, stress disorders, anxiety, major depressive disorders, major depressive disorders with anxiety, depression, sunburn; sexual dysfunction, bipolar disorders, substance use disorders, schizophrenic disorders, movement disorders, cognitive disorders, and diseases, disorders and adverse conditions caused by *Helicobacter pylori*, in a mammal especially a human, comprising an amount of the compound of Formula (I), or a pharmaceutically acceptable salt thereof, that is effective in treating such disorder or condition, and a pharmaceutically acceptable carrier.

The invention also relates to the use of a compound of Formula (I), or a pharmaceutically acceptable salt thereof for the manufacture of a medicament for treating a disorder or condition selected from cardiovascular diseases, allergic disorders, angiogenesis, gastrointestinal disorders, central nervous system disorders, inflammatory diseases, emesis, urinary incontinence, pain, migraine, severe anxiety disorders, stress disorders, anxiety, major depressive disorders, major depressive disorders with anxiety, depression, sunburn, sexual dysfunction, bipolar disorders, substance use disorders, schizophrenic disorders, movement disorders, cognitive disorders, and diseases, disorders and adverse conditions caused by *Helicobacter pylori,*

The term "treating" as used herein refers to reversing, alleviating, inhibiting the progress of, or preventing the disorder or condition to which such term applies, or one or more symptoms of such disorder or condition. The term "treatment" as used herein refers to the act of treating, as "treating" is defined immediately above.

### Detailed Description of the Invention

In this specification, the term "halo" means F, Cl, Br and I, preferably Cl or F.

The term "alkyl" as used herein refers to straight or branched chain saturated radicals, including, but not limited to methyl, ethyl, *n*-propyl, *iso*propyl, and t-butyl.

The term "halo C₁-C₆ alkyl" is used herein to mean a straight, branched or cyclic C₁-C₆ alkyl substituted by one or more (preferably one to seven) halo. These compounds include, but are not limited to, trifluoromethyl, difluoroethyl, trifluoroethyl, pentafluoroethyl, trifluoroisopropyl, tetrafluoroisopropyl, pentafluoroisopropyl, hexafluoroisopropyl, and the like.

The compounds of Formula (I) contain at least two chiral centers and therefore exist as at least two diastereoisomeric pairs of optical isomers including epimers. This invention includes both the individual isomers of the compounds of Formula (I) together with mixtures thereof.

A preferred group of compounds of Formula (I) is that wherein R¹ is C₁-C₃ alkyl; R² is hydrogen, C₁-C₃ alkyl, halo C₁-C₃ alkyl or phenyl; R³ is hydrogen or fluorine; R⁴ and R⁵ are independently hydrogen, C₁-C₃ alkyl or halo C₁-C₃ alkyl; and n is one or two.

A more preferred group of compounds of Formula (I) is that wherein R¹ is methyl; R² is hydrogen, methyl, trifluoromethyl or phenyl; R³ is hydrogen; and R⁴ and R⁵ are hydrogen.

The compounds of Formula (I) preferably have (2*S*,3*S*)- configuration with the piperidine ring.

Preferred individual compounds are:
(2*S*,3*S*)-3-(6-methoxy-3-trifluoromethyl-1,3-dihydroisobenzofuran-5-yl)methylamino-2-phenylpiperidine or its salts;
(2*S*,3*S*)-3-(6-methoxy-1 -methyl-1 -trifluoromethylisochroman-7-yl)methylamino-2-phenylpiperidine or its salts;
(2*S*,3*S*)-3-(6-methoxy-3-methyl-3-trifluoromethyl-1,3-dihydroisobenzofuran-5-yl)methylamino-2-phenylpiperidine or its salts;
(2*S*,3*S*)-3-(6-methoxy-3 -phenyl-3-trifluoromethyl-1,3-dihydroisobenzofuran-5-yl)methylamino-2-phenylpiperidine or its salts; and
(2S,3 S)-3-[1-(6-methoxy-3-methyl-3-trifluoromethyl-1,3-dihydroisobenzofuran-5-yl)ethylamino]-2-phenylpiperidine or its salts.

Particularly preferred individual compounds are (2*S*,3*S*)-3-[(1R)-6-methoxy-1-methyl-1-trifluoromethylisochroman-7-yl]methylamino-2-phenylpiperidine or its salts, and (2S,3S)-3-[(3R)-6-methoxy-3-methyl-3-trifluoromethyl-1,3-dihydroisobenzofuran-5-yl)methylamino-2-phenylpiperidine or its salts.

Further, the present invention provides a compound of Formula (III): 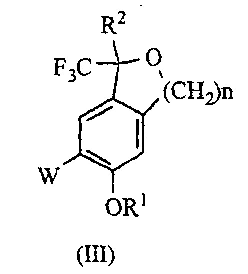 wherein W is hydrogen or Q(O=)C- wherein Q is H, C₁-C₆ alkyl or halo C₁-C₆ alkyl; R¹ is C₁-C₆ alkyl (preferably methyl); R² is hydrogen, C₁-C₆ alkyl, halo C₁-C₆ alkyl or phenyl (preferably hydrogen, methyl, trifluoromethyl or phenyl); and n is one, two or three (preferably one or two).

These compounds of Formula (III) can be used as intermediates to prepare the compounds of Formula (I). The compounds of Formula(III) contain one chiral center. Therefore, this invention also includes both individual isomers of the compounds of Formula (III) together with mixture thereof.

Preferred compounds of Formula (III) include enantiomeric pairs of
5-methoxy- 1 -trifluoromethyl-1,3-dihydroisobenzofuran;
6-methoxy-3-trifluoromethyl-1,3-dihydrobenzofuran-5-carbaldehyde;
5-methoxy-1,1 -bistrifluoromethyl-1,3-dihydroisobenzofuran;
6-methoxy-3,3-bis(trifluoromethyl)-1,3-dihydroisobenzofuran-5-carbaldehyde;
6-methoxy-1-methy]-1-trifluoromethylisochroman;
6-methoxy-1-methyl-1-trifluoromethylisochroman-7-carbaldehyde;
5-methoxy-1-methyl-1 -trifluoromethyl-1,3-dihydroisobenzofuran;
6-methoxy-3-methyl-3-trifluoromethyl-1,3-dihydroisobenzofuran-5-carbaldehyde;
1-trifluoromethyl-5-methoxy-1-phenyl-1,3-dihydroisobenzofuran;
3-trifluoromethyl-6-methoxy-3-phenyl-1,3-dihydroisobenzofuran-5-carbaldehyde; and
5-acetyl-3-methyl-6-methoxy-3-trifluoromethyl-1,3-dihydroisobenzofuran.

Particularly preferred compounds of Formula (III) include:
(1R)-6-methoxy-1-methyl-1-trifluoromethylisochroman;
(1R)-6-methoxy-1 -methyl-1 -trifluoromethylisochroman-7-carbaldehyde;
(1S)-6-methoxy-1-methyl-1-trifluoromethylisochroman;
(1S)-6-methoxy-1-methyl-1-trifluoromethylisochroman-7-carbaldehyde;
(1R)-5-methoxy-1-methyl-1-trifluoromethyl-1,3-dihydroisobenzofuran;
(1R)-6-methoxy-3 -methyl-3 -trifluoromethyl-1,3-dihydroisobenzofuran-5-carbaldehyde;
(1S)-5-methoxy-1-methyl-1-trifluoromethyl-1,3-dihydroisobenzofuran; and
(1S)-6-methoxy-3-methyl-3-trifluoromethyl-1,3-dihydroisobenzofuran-5-carbaldehyde.

### General synthesis:

The piperidinylaminomethyl trifluoromethyl cyclic ether compounds of Formula (I) of this invention may be prepared as described in the following reaction schemes.

Unless otherwise indicated, in the reaction schemes that follow, R¹, R², R³, R⁴, R⁵, Q and n are defined as above, and Z represents hydrogen or amino protecting group.

Scheme 1 illustrates a method for preparation of a compound of Formula (Ia) by reductive alkylation of Compound (II) with Compound (III).

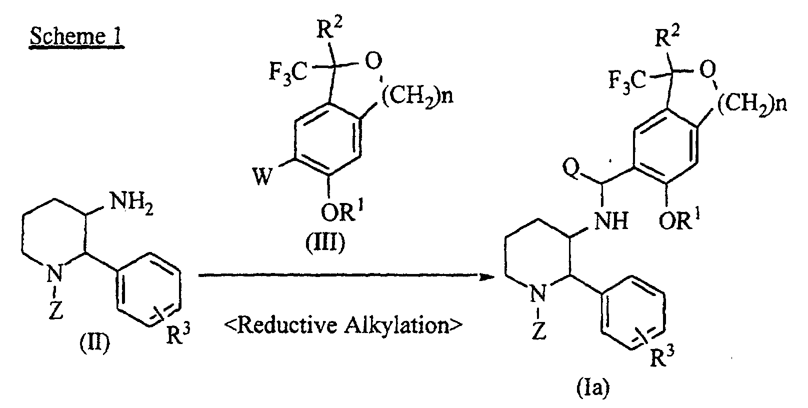

A compound of Formula (Ia) wherein Z is hydrogen or an amino protecting group, can be synthesized by reductive alkylation of an amine compound of Formula (II) with a compound of Formula (III) according to the known procedures as described in the International Patent Publication No. WO 97/03066. The reaction can be carried out in the presence of a suitable reducing reagent in a reaction inert solvent. The suitable reducing reagents are, for example, borohydrides such as sodium triacetoxyborohydride (NaB(OAc)₃H), sodium borohydride (NaBH₄) and sodium cyano borohydride (NaBH₃CN), boranes, lithium aluminum hydride (LiAlH₂), and trialkylsilans. The suitable solvents include polar solvents such as methanol, ethanol, methylene chloride, tetrahydrofuran (THF), dioxane and ethylacetate. The reaction can be conducted at from about -78°C to the reflux temperature of the solvent, preferably from 0 to 25°C for 5 minutes to 48 hours, preferably 0.5 to 12 hours. Preferably, Compounds (Ia) wherein Q is other than hydrogen can be obtained by reacting Compound (II) with Compound (III) wherein W is an acyl group. This reaction can be carried out in the presence of a reducing agent such as NaBH₃CN and a Lewis acid such as tin(IV) chloride (TiCl₄) in a reaction inert solvent such as dichloromethane (*Tetrahedron Letter,* Vol. 31, p. 5547, 1990). When Z is an amino protecting group, the amino protecting group can be removed after the reductive alkylation using methods known to a person skilled in the art (see, e.g., *Protective Groups in Organic Synthesis, T.* W. Greene, *et al*., John Wieley & Sons, Inc., 1991), to obtain the compound of Formula (I). Specifically, when Z is *tert-*butoxycarbonyl (abbreviated as "Boc"), Boc can be removed in the presence of an acid such as HCl in a reaction inert solvent such as methanol under an inert atmosphere (e.g., under nitrogen atmosphere).

A starting material of Formula (II) can be prepared by nitrogen protection of a_(2S,3S)-3-amino-2-phenylpiperidine compound, which can be prepared by the known methods as described, for example, in the International Patent Publication No. WO 92/17449. The nitrogen protection of the piperidine ring of the compounds of Formula (II) can be carried out according to known procedures as described in, for example, the International Patent Publication No. WO 97/03066. Suitable protecting group are for example Boc, benzyloxycarbonyl chloride (abbreviated as Cbz) or trifluoroacetyl. For example, nitrogen protection by Boc can be carried out by treating the (2S,3S)-3-amino-2-phenylpiperidine compound with (t-BuOCO)₂O in the presence of a base such as sodium hydroxide, sodium bicarbonate or triethylamine.

Compounds of Formula (III) can be prepared by formylation or acylation of compounds of Formula (IV) as illustrated in Scheme 2.

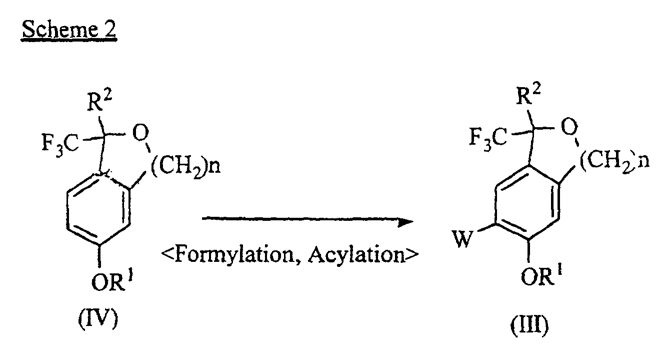

Known formylation or acylation methods can be used. For example, direct formylation may be accomplished by contacting Compound (IV) with a suitable formylating agent in the presence of a suitable catalyst. Suitable formylating agent/catalyst systems includes dichloromethyl methyl ether/titanium (IV) chloride (CH₂CHOCH₃/TiCl₄), trifluoroacetic acid (CF₃CO₂H)/hexamethylenetetramine (modified Duff's conditions) and phosphoryl trichloride (POCl₃)/DMF (Vilsmeier's conditions). More specifically, the formylation of Compound (IV) with CH₂CHOCH₃/TiCl₄ can be carried out in a reaction inert solvent under nitrogen atmosphere. The suitable solvents includes dichloromethane and 1,2-dichloroethane at from about -120°C to room temperature for about 1 minute to 10 hours, preferably -78°C for 5 minutes to 4 hours. The Duff reaction can be also applied to the formylation in accordance with the reaction conditions disclosed in International Patent Publication WO 94/24081.

Also, a suitable indirect formylation method comprises (i) halogenating Compound (IV), (ii) replacing the halogen atom by a cyano group, and then (iii) subjecting the resultant cyano-substituted compound to reduction. (i) The halogenation may be carried out according to the known procedures as reported by G. A. Olah *et al*. (*J*. *Org*. *Chem.,* Vol. 58, pp. 3194-, 1983). (ii) The replacement of the halogen atom with a cyano group can be achieved according to the known procedures as reported by D. M. Tschaem *et al*., (*Synth. Commun.,* Vol. 24, pp. 887-, 1994) or by K. Takagi *et al*., (*Bull*. *Chem*. *Soc*. *Jpn.,* Vol 64, pp. 1118-, 1991). (iii) The reduction as used herein may be achieved in the presence of diisopropyl aluminiumhydride (DIBAL-H) in dichloromethane or Raney Ni in formic acid.

The acylation can be achieved by well-known Friedel-Crafts acylation described for example in *Advanced Organic Chemistry* by Jerry March, John Wiely & Sons, forth edition, 1992, p. 539, and the references therein. More specifically, Compound (IV) can be reacted with an acylating agent in the presence of an acid catalyst to give Compound (III). Suitable acylating agents include acyl chloride, acyl fluoride and anhydrides, preferably acyl chloride. Suitable acid catalysts include sulfuric acid and Lewis acid such as aluminum chloride, preferably aluminum chloride. This reaction can typically be carried out at a temperature from about -10°C to room temperature, for about 5 minutes to 2 hours, preferably at about 0°C for about 1 hour.

A cyclic ether of Formula (IV) can be prepared from a compound of Formulae (Va) or (Vb) according to the known procedures as reported by W. E. Parham *et al*. (*J*. *Org. Chem.,* Vol. 39, pp. 2048, 1974) or the procedures illustrated in Scheme 3.

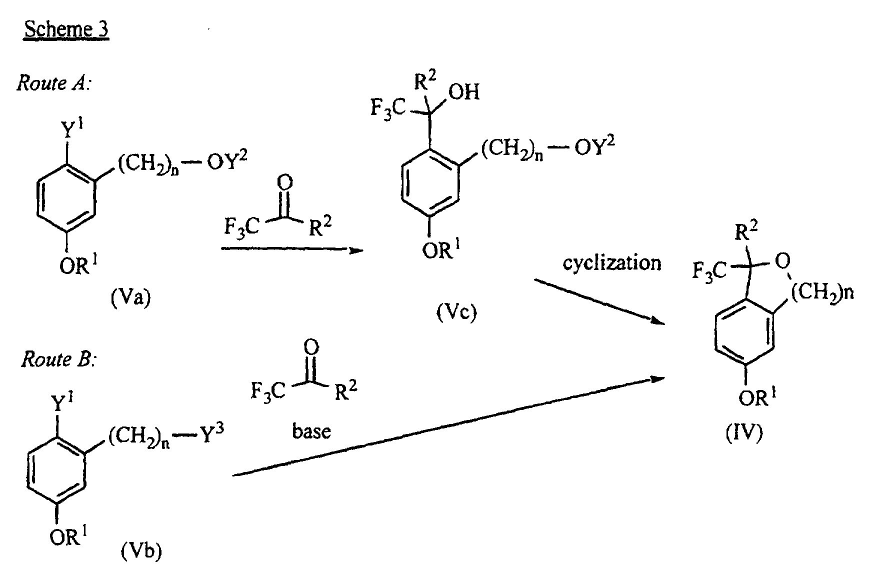

In Route A of Scheme 3, a compound of Formula (IV) can be synthesized from a compound of Formula (Va) wherein Y¹ is Br, I or Cl (preferably Br) and Y² is hydrogen or a hydroxy protecting group (suitably tetrahydropyranyl, abbreviated as "THP"). The compound of Formula (Va) can be metallated by treatment with an organometallic compound. The reaction mixture can subsequently be treated with a carbonyl compound represented by CF₃C(=O)R² to give the diol (Vc). If required, the hydroxy protecting group Y² of the diol (Vc) can be removed. Then, the diol (Vc) can be subjected to cyclization to give the cyclic ether compound (IV).

The metallation of compound (Va) can be carried out in the presence of an organometallic compounds such as n-buthyllithium, sec-buthyllithium or *tert*-buthyllithium. The metallation and the subsequent reaction with CF₃C(=O)R² can be carried out in a reaction inert solvent such as THF, ether and hexane under an inert atmosphere, for example, under nitrogen, at from about -150°C to room temperature for 15 minutes to 12 hours, preferably from -120°C to -30°C for 10 minutes to 6 hours. The hydroxy protection and deprotection with a protecting group Y² can be achieved under suitable conditions depending on the protecting group chosen according to known methods (see for example *Protecting Group in Organic Synthesis* by T. W. Greene *et al.,* published from John Wiely & Sons, Inc.).

The cyclization of the diol (Vc) can be carried out in the presence of an acid according to the known methods reported as by for example W. E. Parham *et al. (Synthesis,* pp. 116-, 1976) or D. Seebach *et al*. (*Chem*. *Ber*., Vol. 116, pp. 8354-, 1994). Suitable acids are, for example, HCI, H₂SO₄ or *p*-toluenesulfonic acid trifluoro acetic acid (abbreviated as TFA). The reaction can be carried out at from about room temperature to about 200°C for 10 minutes to 12 hours, preferably at 60°C to 150°C for 30 minutes to 6 hours.

Alternatively, the cyclization can be carried out according to the procedures known as Mitsunobu reaction or the procedures reported by J. R. Falck *et al*. (*J*. *Am*. *Chem*. *Soc.,* Vol. 116, pp. 8354-, 1994). For example, the Mitsunobu reaction can be carried out in the presence of triphenyl phosphine/diethyl azodicarboxylate in a suitable solvent such as dichloromethane under nitrogen at about 0°C for from about 5 minutes to 6 hours.

In Route B of the Scheme 3, a cyclic ether compound of Formula (IV) can be synthesized by subjecting a compound of Formula (Vb), wherein Y³ is a leaving group, to a one-step cyclization with CF₃C(=O)R² in the presence of a suitable base (see for example *J*. *Org*. *Chem.,* Vol. 41, pp. 1184-, 1976). Suitable leaving groups include Cl, Br, tosylate, mesylate and triflate. Suitable bases include alkyllithium such as n-BuLi, sec-BuLi or t-BuLi. For example, the reaction can be carried out first by treating a compound of Formula (Vb) with n-BuLi in a suitable reaction inert solvent such as THF/hexane, under nitrogen at from about -120°C to 0°C for about 5 minutes to 12 hours, preferably -100°C to -60°C for 10 minutes to 6 hours. Subsequently, to the reaction mixture the carbonyl compound CF₃C(=O)R² can be added and the temperature can be elevated to about -50°C to room temperature.

On the other hand, for example, a starting material of Formulae of (Va) and (Vb), wherein R¹ is methyl, can be prepared by bromination at the para position of a known or commercially available anisole compound according to known methods (e.g., *J*. *Org. Chem.,* Vol. 58, pp. 7507-, 1993, and *J. Org. Chem.,* Vol. 46, pp.118-, 1981).

Unless indicated otherwise, the pressure of each of the above reactions is not critical. Generally, the reactions will be conducted at a pressure of about one to about three atmospheres, preferably at ambient pressure (about one atmosphere).

The compounds of Formula (I), and the intermediates shown in the above reaction schemes can be isolated and purified by conventional procedures, such as recrystallization or chromatographic separation.

As the piperidinylaminomethyl trifluoromethyl cyclic ether compounds of this invention possess at least two asymmetric centers, they are capable of occurring in various stereoisomeric forms or configurations (e.g., diastereoisomers including epimers). Hence, the compounds can exist in separated (+)- and (-)-optically active forms, as well as mixtures thereof. The present invention includes all such forms within its scope. All optical isomers and stereoisomers of the compounds of formula (I), (II) and mixture thereof, are considered to be within the scope of the invention. With respect to the compounds of formula(I) and (II), the invention includes the use of racemate, one or more enantiomeric forms, one or more diastereomeric forms, or mixture thereof. The compounds of formula (I) and (II) may also exist as tautomers. This invention relates to use of all such tautomers and mixtures thereof. Individual isomers can be obtained by known methods, such as optical resolution, fractional crystallization, chromatography or H.P.L.C. of a diastereomeric mixture of an intermediate, or a compound of Formula (I) or a suitable salt thereof. Also, the individual stereoisomers can be synthesized from the appropriate optically active starting materials or intermediates using any of the general processes described herein.

In so far as the piperidinylaminomethyl trifluoromethyl cyclic ether compounds of this invention are basic compounds, they are all capable of forming a wide variety of different salts with various inorganic and organic acids. Although such salts must be pharmaceutically acceptable for administration to animals, it is often desirable in practice to initially isolate the base compound of this invention from the reaction mixture as a pharmaceutically unacceptable salt and then simply convert to the free base compound by treatment with an alkaline reagent and thereafter convert the free base to a pharmaceutically acceptable acid addition salt. The acid addition salts of the base compounds of this invention are readily prepared by treating the base compound with a substantially equivalent amount of the chosen mineral or organic acid in an aqueous solvent or in a suitable organic solvent, such as methanol or ethanol. Upon careful evaporation of the solvent, the desired solid salt is readily obtained. The acids which are used to prepare the pharmaceutically acceptable acid addition salts of the aforementioned base compounds of this invention are those which form non-toxic acid addition salts, i.e., salts containing pharmaceutically acceptable anions, such as the hydrochloride, hydrobromide, hydroiodide, nitrate, sulfate or bisulfate, phosphate or acid phosphate, acetate, lactate, citrate or acid citrate, tartrate or bi-tartrate, succinate, maleate, fumarate, gluconate, saccharate, benzoate, methanesulfonate, ethanesulfonate, benzenesulfonate, p-toluenesulfonate and pamoate (i.e., 1.1'-methylene-bis-(2-hydroxy-3-naphthoate) salts).

The piperidinylaminomethyl trifluoromethyl cyclic ether compounds of the present invention exhibit significant substance P receptor-binding activity and therefore are of value in the treatment of a wide variety of clinical conditions which are characterized by the presence of an excess of said substance P activity. Such conditions include cardiovascular diseases, allergic disorders, angiogenesis, gastrointestinal disorders, central nervous system disorders, inflammatory diseases, emesis, urinary incontinence, pain, migraine; severe anxiety disorders, stress disorders, anxiety, major depressive disorders, major depressive disorders with anxiety, depression, sunburn; sexual dysfunction, bipolar disorders, substance use disorders, schizophrenic disorders, movement disorders, cognitive disorders, and diseases, disorders and adverse conditions caused by *Helicobacter pylori,* in a mammal, especially humans. For treatment of emesis, these compounds may preferably be used in combination with a 5HT₃ receptor antagonist.

The active piperidinylaminomethyl trifluoromethyl cyclic ether compounds of Formula (I) of this invention, or their pharmaceutically acceptable salts, can be administered via either the oral, parenteral (e.g., intravenously, intramuscularly or subcutaneously) or topical routes to mammals. In general, these compounds are most desirably administered to humans in doses ranging from about 0.3 mg up to 750 mg per day, although variations will necessarily occur depending upon the weight and condition of the subject being treated and the particular route of administration chosen. However, a dosage level that is in the range of from about 0.06 mg to about 6 mg per kg of body weight per day is most desirably employed.

Nevertheless, variations may still occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effects provided that such higher dose levels are first divided into several small doses for administration throughout the day.

The piperidinylaminomethyl trifluoromethyl cyclic ether compounds of the present invention may be administered alone or in combination with pharmaceutically acceptable carriers or diluents by any of the above routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media end various nontoxic organic solvents, etc. Moreover, oral pharmaceutical compositions can be suitably sweetened and/or flavored. In general, the therapeutically-effective compounds of this invention are present in such dosage forms at concentration levels ranging about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art.

Additionally, it is also possible to administer the compounds of the present invention topically when treating, for example, inflammatory conditions of the skin and this may preferably be done by way of creams, jellies, gels, pastes, ointments and the like, in accordance with standard pharmaceutical practice.

The activity of the compounds of the present invention, as substance P antagonists, can be determined by their ability to inhibit the binding of substance P at its receptor sites in CHO-cells which express NK1 receptor or IM-9 cells employing radioactive reagents. The substance P antagonist activity of the herein described piperidinylaminomethyl trifluoromethyl cyclic ether compounds can be evaluated by using the standard assay procedure described by D. G. Payan *et al*., (*The Journal of Immunology,* Vol. 133, p. 3260, 1984). This method essentially involves determining the concentration of the individual compound required to reduce by 50% the amount of radiolabelled substance P (SP) reagents at their receptor sites in said isolated cow tissues or IM-9 cells, thereby affording characteristic IC₅₀ values for each compound tested. More specifically, inhibition of [³H]SP binding to human IM-9 cells by compounds are determined in assay buffer (50 mM Tris-HCl (pH 7.4), 1 mM MnCl₂, 0.02 % bovine serum albumin, bacitracin (40 µg/ml), leupeptin (4 µg/ml), chymostatin (2 µg/ml) and phosphoramidon (30 µg/ml)). The reaction is initiated by the addition of cells to assay buffer containing 0.56 nM [³H]SP and various concentrations of compounds (total volume; 0.5 ml) and incubation for 120 min at 4 °C. Incubation is terminated by filtration onto GF/B filters (presoaked in 0.1 % polyethylenimine for 2 hours). Nonspecific binding is defined as the radioactivity remaining in the presence of 1 µM SP. The filters are placed into tubes and counted using a liquid scintillation counter.

Alternatively, the anti-inflammatory activity of the compounds of this invention, in the periphery of a mammalian subject, is demonstrated by a capsaicin-induced plasma extravasation test, using the procedure described by A. Nagahisa *et al*, (*European Journal of Pharmacology,* Vol. 217, pp. 191-195, 1992). In this test, anti-inflammatory activity is determined as the percent inhibition of plasma protein extravasation in the ureter of pentobarbital-anesthetized (25 mg/kg i.p.) male Hartey guinea pigs (weighing 300-350 g). Plasma extravasation is induced by intraperitoneal injection of capsaicin (30 µM in 0.1 BSA containing buffer, 10 ml/animal) into the animals, which are fasted overnight. The compounds of this invention were dissolved in 0.1 % methyl cellulose-water and given orally 1 hour before capsaicin challenge. Evans blue dye (30 mg/kg) was administered intravenously 5 minutes before challenge. The animals were killed 10 minutes after capsaicin injection and both right and left ureter were removed. Tissue dye content was quantitated at 600 nm absorbance after overnight formamide extraction.

The compound prepared in Example 3 of this invention showed 98 % inhibition at 0.03 mg/kg, while the structurally closest compound in Example 18 of WO 97/08114 showed 72 % at the same dosage.

The adverse effect on Ca²⁺ channel binding affinity is determined by study of verapamil binding in a rat heart membrane preparation. More specifically, verapamil binding is performed as previously described by Reynolds *et al*., (*J*. *Pharmacol*. *Exp*. *Ther.* Vol. 237, p. 731, 1986). Briefly, incubations are initiated by the addition of tissue to tubes containing 0.25 nM [³H]desmethoxyverapamil and various concentrations of compounds (total volume 1 ml). Nonspecific binding is defined as radioligand binding remaining in the presence of 3-10 µM methoxyverapamil.

The activity of the compounds of this invention against CNS disorders is determined in a [Sar⁹, Met(O₂)¹¹]substance P-induced tapping test in gerbils using a modification of the method ofN. M. J Rupniak (*European Journal of Pharmacology,* Vol. 265, pp. 179-183, 1994) and L. J. Bristow (*European Journal of Pharmacology,* Vol. 253, pp. 245-252, 1994). More specifically, first a compound of this invention is subcutanously administered into a gerbil. Second, gerbils are lightly anesthetized with ether and the skull surface is exposed. Third, [Sar⁹, Met(O₂)¹¹]substance P (5 µl) are administered directly into the lateral ventricles via a 25 gauge needle inserted 3.5 mm below lambda. Then, gerbils are placed individually in 1 liter beakers and monitored for repetitive hind paw tapping.

Anti-emetic activity of the compounds of this invention can be demonstrated in cisplatin-induced emesis test in ferrets. A compound of this invention is subcutaneously administered to the ferrets (male, b.w.=1.3-1.6 kg) 30 minutes before cisplatin injections. Cisplatin is intraperitoneally injected to the ferrets, and their emetic episodes (i.e., retching, vomiting and gagging) are recorded by a video camera for 4 hours. The frequencies of the episodes are counted.

Some compounds of this invention exhibited good anti-emetic activity in the tests (ED₉₀ of 0.05 mg/kg to 0.1 mg/kg).

The susceptibility to metabolism of the compounds of this invention can be evaluated by an in-vitro assay that comprises (a) contacting a sample compound with a reagent composition prepared by adding a specific cytochrome P-450 (e.g., CYP2D6) isozyme to poor metabolizer (abbreviated as PM) liver microsomes (i.e., liver microsomes of a human lacking said specific cytochrome P-450 isozyme) in a carrier material, and (b) analyzing the substrate by a mass spectrometer linked with a HPLC (high performance liquid chromatography). More specifically, the substrate (1 µM) is incubated with PM human liver microsome (manufactured by Keystone Skin Bank) supplemented with a recombinant CYP2D6-expressing microsome (0-0.1 mg/ml) or control vector microsomes in the presence of 1.3 mM NADP (nicotinamide adenine dinucleotide phosphate), 0.9 mM NADH (reduced nicotinamido adenine dinucleotide), 3.3 mM MgCl₂ and 8 units/ml G-6-PDH (glucose-6-phosphate dehydrogenase) respectively in a total volume of 1.2 ml of 100 mM potassium phosphate buffer. The pH of the solution is 7.4, and the incubation temperature is 37 °C. At specific incubation times (0, 5, 10, 30 and 60 minutes), an aliquot of 100 µl is withdrawn from the reaction mixture and mixed with 1 ml of acetonitrile (ACN) containing 5 ng/ml (2S,3S)-3-(2-methoxybenzylamino)-2-diphenylmethyl-1-azabicyclo[2.2.2]octane as an internal standard (prepared according to the procedures disclosed in WO 90/05729). Protein is subsequently precipitated by centrifugation (1,800 x g for 10 min), and the resulting supernatant is taken. Concentration of substrates and products in the sample solutions are analyzed with a Sciex API-III mass spectrometer linked with a Hewlett-Packard HP1090 HPLC system. Concentrations of the remaining substrates in each sample solution (%-remaining) are plotted against the desired incubation times. The values of T_{1/2} are obtained in each graph. The ratios of the T_{1/2} values of the compound tested are calculated (i.e., T_{1/2} ratio=(T_{1/2} by control vector microsome)/(T_{1/2} by PM human liver microsome supplemented CYP2D6-expressing microsome)).

Some compounds prepared in the following working examples exhibited lower susceptibility to metabolism comparing to the structurally closest compounds of International Patent Publication WO 97/08144.

### EXAMPLE

The present invention is illustrated by the following examples. However, it should be understood that the invention is not limited to the specific details of these examples. Melting points were taken with a Buchi micro melting point apparatus and not corrected. Infrared absorption spectra (IR) were measured by a Shimadzu infrared spectrometer (IR-470). ¹H nuclear magnetic resonance spectra (NMR) was measured in CDCl₃ by a JEOL NMR spectrometer (JNM-GX270, 270MHz for ¹H) unless otherwise indicated and peak positions are expressed in parts per million (ppm) downfield from tetramethylsilane. The peak shapes are denoted as follows: s, singlet; d, doublet; t, triplet; m, multiplet.

### Example 1

### Preparation of (2S,3S)-3-(6-Methoxy-3-trifluoromethyl-1,3-dihydroisobenzofuran-5-yl)methylamino-2-phenylpiperidine dihydrochloride

### (i) 2-Bromo-5-methoxybenzylalcohol

To a stirred suspension of lithium aluminum hydride (1.2 g, 30.6 mmol) in dry tetrahydrofuran (40 ml) was added a solution of methyl 2-bromo-5-methoxybenzoate (5.0 g, 20.4 mmol) in dry tetrahydrofuran (80 ml) dropwise under nitrogen at 0°C. The reaction mixture was stirred at 0°C for I hr. To the reaction mixture was added sodium sulfate decahydrate and potassium fluoride. The resulting mixture was stirred at room temperature for 1 hr, and filtered through a celite pad. The filtrate was concentrated to give crude products as a white crystal. This was purified by silica-gel column chromatography eluted with a gradient of hexane and ethyl acetate (10 : 1, 8 : 1, 6 : 1) to give the title compound as a white crystal (4.2 g, 94.9%).
¹H-NMR(CDCl₃) : 7.42 (d, J = 8.8 Hz, 1H), 7.07 (d, J = 2.9 Hz, 1H), 6.72 (dd, J = 8.8,2.9 Hz, 1H), 4.71 (d, J = 6.2 Hz, 2H), 3.81 (s, 3H), 1.98 (t, J = 6.2 Hz, 1H)

### (ii) 2-(2-Bromo-5-methoxybenzyloxy)tetrahydropyran

To a stirred mixture of 2-bromo-5-methoxybenzylalcohol (3.91 g, 18.0 mmol) and dihydropyran (3.3 ml, 36.0 mmol) in dry dichloromethane (80 ml) was added camphor sulfonic acid (210 mg, 0.9 mmol) under nitrogen at 0°C. The reaction mixture was stirred at 0°C for 1 hr. The reaction mixture was quenched with saturated aqueous sodium bicarbonate solution, and extracted with dichloromethane. The organic extracts were washed with brine, dried over magnesium sulfate, and concentrated to give a crude product. This was purified by silica-gel column chromatography eluted with a mixed solvent of hexane and ethyl acetate (20 : 1) to give the title compound as a colorless oil (5.68 g, quant.).
¹H-NMR(CDCl₃) : 7.40 (d, J = 8.8 Hz, 1H), 7.10 (d, J = 3.3 Hz, 1H), 6.69 (dd, J = 8.8, 3.3 Hz, 1H), 4.80 - 4.76 (m, 2H), 4.53 (d, J = 13.6 Hz, 1H), 3.97 - 3.88 (m, 1H), 3.79 (s, 3H), 3.60 - 3.53 (m, 1H), 1.93 - 1.54 (m, 6H)

### (iii) 2,2,2-Trifluoro-1-(4-methoxy-2-(tetrahydropyran-2-yloxymethyl)phenyl)ethanol

To a stirred solution of 2-(2-bromo-5-methoxybenzyloxy)tetrahydropyran (1.0 g, 3.32 mmol) in dry tetrahydrofuran (20 ml) was added n-butyllithium (2.6 ml, 4.32 mmol) dropwise under nitrogen at -78°C. The reaction mixture was stirred at -40°C for 2.5 hr. To the reaction mixture was added a solution of trifluoromethylacetaldehyde (0.7 ml) in dry tetrahydrofuran (2 ml) dropwise at -78°C. After 2 hr at the same temperature, the reaction was quenched by saturated aqueous ammonium chloride, and extracted with dichloromethane. The organic layers were dried over magnesium sulfate and concentrated to give crude products, which were purified by silica-gel column chromatography eluted with gradient of hexane and ethyl acetate (30 : 1, 20 : 1, 10 : 1, 6 : 1, 5 : 1) to give the title compound as a colorless oil (390 mg, 36.7%).
¹H-NMR(CDCl₃) : 7.53 (d, J = 8.4 Hz, 1H), 6.94 (d, J = 2.9 Hz, 1H), 6.89 (dd, J = 8.4, 2.9 Hz, 1H), 5.36 - 5.25 (m, 1H), 4.85 and 4.78 (each d, J = 12.1 Hz, total 1H), 4.69 - 4.63 (m, 1H), 4.58 and 4.51 (each d, J = 12.1 Hz, total 1H), 3.88 - 3.70 (m, 2H), 3.81 (s, 3H), 3.56 - 3.51 (m, 1H), 1.85 - 1.50 (m, 6H)

### (iv) 2,2,2-Trifluoro-1-(2-hydroxymethyl-4-methoxyphenyl)ethanol

A mixture of 2,2,2-trifluoro-1-(4-methoxy-2-(tetrahydropyran-2-yloxymethyl)phenyl)ethanol (390 mg, 1.22 mmol) and a mixed solvent of acetic acid : tetrahydrofuran : water (4 : 2 : 1, 24 ml) was stirred at room temperature for 2 hr. The reaction temperature was allowed to elevate, and kept at 40°C for 1.5 hr, and then at 60°C for 2 hr. The solvent was removed, and the residue was dried under vacuum to give crude material of the tittle compound as a slight yellow oil (410 mg). This material was used without further purification.

### (v) 5-Methoxy-1-trifluoromethyl-1,3-dihydroisobenzofuran

To a stirred and ice-cooled solution of 2,2,2-trifluoro-1-(2-hydroxymethyl-4-methoxyphenyl)ethanol (160 mg, 0.54 mmol) and triphenylphosphine (312 mg, 1.19 mmol) in dry dichloromethane (6 ml) was added a solution of diethyl azodicarboxylate (0.255 ml, 1.62 mmol) in dry dichloromethane (2 ml) dropwise under nitrogen. The yellow reaction mixture was stirred at 0°C for 30 min, and then at room temperature for 2 hr. Dichloromethane and water were added to the reaction mixture, and the aqueous layer was extracted with dichloromethane. The extracts were combined and concentrated to give a crude product, which was purified by silica-gel column chromatography eluted with hexane : ethyl acetate (100:1 to 20:1) to give the title compound as a colorless oil (67 mg, 56.9%).
¹H-NMR(CDCl₃) : 7.29 (d, J = 8.4 Hz, 1H), 6.88 (dd, J = 8.4, 2.2 Hz, 1H), 6.80 (br.s, 1H), 5.42 - 5.39 (m, 1H), 5.28 - 5.12 (m, 2H), 3.83 (s, 3H)

### (vi) 6-Methoxy-3-trifluoromethyl-1,3-dihydroisobenzofuran-5-carbaldehyde

To a stirred solution of 5-methoxy-1-trifluoromethyl-1,3-dihydroisobenzofuran (67 mg, 0.31 mmol) in dry dichloromethane (5 ml) was added titanium (IV) chloride (0.074 ml, 0.68 mmol) under nitrogen at -78°C. After 15 min, to the yellow solution was added a solution of dichloromethyl methyl ether (0.056 ml, 0.61 mmol) in dry dichloromethane (1 ml) at same temperature. The reaction mixture was stirred at -78°C for 1 hr, poured onto ice-water, and stirred at room temperature for 30 min. The aqueous layer was extracted with methylene chloride. The extracts were washed with brine, dried over magnesium sulfate, and concentrated to give a crude product. This was purified by silica-gel column chromatography eluted with a gradient of hexane and ethyl acetate (10 : 1, 8 : 1, 6 : 1) to give the title compound as a white crystal (63 mg, 82.5%).
¹H-NMR(CDCl₃) : 10.45 (s, 1H), 7.87 (s, 1H), 6.92 (s, 1H), 5.46 - 5.39 (m, 1H), 5.30 (dd, J = 13.9, 2.2 Hz, 1H), 5.19 (d, J = 13.9 Hz, 1H), 3.97 (s, 3H)

### (vii) 1-tert-Butoxycarbonyl-(2S,3S)-3-(6-methoxy-3-trifluoromethyl-1,3-dihydroisobenzofuran-5-yl)methylamino-2-phenylpiperidine

To a stirred solution of 1-tert-butoxycarbonyl-(2S,3S)-3-amino-2-phenylpiperidine (71 mg, 0.26 mmol) which was prepared by a method described in International Patent Publication WO 97/03066, and 6-methoxy-3-trifluoromethyl-1,3-dihydroisobenzofuran-5-carbaldehyde (63 mg, 0.26 mmol) in dry dichloromethane (3 ml) was added sodium triacetoxyborohydride (76 mg, 0.36 mmol) portionwise under nitrogen at room temperature. The reaction mixture was stirred at room temperature for 5 hr. The pH was adjusted to below 10 with a saturated sodium bicarbonate solution, extracted with dichloromethane, dried over magnesium sulfate, and concentrated to give a crude product. This was purified by silica-gel column chromatography eluted with a gradient of dichloromethane and methanol (50 : 1, 25 : 1, 20 : 1) to give the title compound as a white amorphous solid (130 mg, 98.7%).
¹H-NMR(CDCl₃); 7.60 - 7.54 (m, 2H), 7.35 - 7.22 (m, 4H), 6.70 (s, 1H), 5.46 - 5.36 (m, 2H), 5.24 and 5.23 (each d, J = 12.1 Hz, total 1H), 5.12 (d, J = 12.1 Hz, 1H), 3.98 - 3.91 (m, 1H), 3.88 - 3.80 (m, 2H), 3.72 (s, 3H), 3.05 - 2.96 (m, 2H), 1.82 - 1.61 (m, 4H), 1.50 - 1.36 (m, 1H), 1.40 (s, 9H)

### (viii) (2S,3S)-3-(6-Methoxy-3-trifluoromethyl-1,3-dihydro-isobenzofuran-5-yl)methylamino-2-phenylpiperidine dihydrochloride

To a stirred solution of 1-tert-butoxycarbonyl-(2S,3S)-3-(6-methoxy-3-trifluoromethyl-1,3 -dihydroisobenzofuran-5-yl)methylamino-2-phenylpiperidine (130 mg, 0.26 mmol) in ethyl acetate (5 ml) was added a methanolic HCI solution (2.5 ml) dropwise under nitrogen at room temperature. The reaction mixture was stirred at room temperature for 8 hr. The solvent was removed, and recrystallized from ethanol to give the title compound as a white crystal (38 mg, 30.5%).
mp: 180-187°C
¹H-NMR(free amine, CDCl₃): 7.28 - 7.25 (m, 5H), 7.01 and 6.96 (each s, total 1H), 6.57 and 6.55 (each s, total 1H), 5.32 - 5.30 (m, 1H), 5.24 - 5.07 (m, 2H), 3.89 (d, J = 2.2 Hz, 1H), 3.70 and 3.64 (each d, J = 13.9 Hz, total 1H), 3.51 - 3.48 (each s, total 3H), 3.40 and 3.38 (each d, J = 13.9 Hz, total 1H), 3.31 - 3.25 (m, 1H), 2.87 - 2.76 (m, 2H), 2.14 - 1.57 (m, 3H), 1.46-1.41 (m, 1H)
Diastereomeric ratio of epimers at 3-position on the dihydroisobenzofuran ring was determined by ¹H-NMR as 5:4.

### Example 2

### Preparation of (2S,3S)-3-(6-Methoxy-3,3-bis(trifluoromethyl)-1,3-dihydroisobenzofuran-5-yl)methylamino-2-phenylpiperidine dihydrochloride

### (i) 1,1,1,3,3,3-Hexafluoro-2-(4-methoxy-2-(tetrahydropyran-2-yloxymethyl)-phenyl)propan-2-ol

To a stirred solution of 2-(2-bromo-5-methoxybenzyloxy)tetrahydropyran (1.0 g, 3.32 mmol) in dry tetrahydrofuran (20 ml) was added n-butyllithium (2.6 ml, 4.32 mmol) dropwise under nitrogen at -78°C. The reaction mixture was stirred at -40°C for 1.5 hr. To the reaction mixture was added a solution of hexafluoroacetone (1 ml) in dry tetrahydrofuran (2 ml) dropwise at -78°C. The resulting mixture was allowed at 0°C for 3 hr. This was quenched by saturated ammonium chloride solution, and extracted with dichloromethane. The organic extracts were dried over magnesium sulfate and concentrated to give a crude product, which was purified by silica-gel column chromatography eluted with gradient of hexane and ethyl acetate (30:1, 25:1, 20:1, 15:1) to give the title compound (890 mg, 69.0%).
¹H-NMR(CDCl₃) : 7.68 - 7.25 (m, 1H), 7.42 (s, 1H), 6.95 - 6.90 (m, 2H), 5.08 (d, J = 11.7 Hz, 1H), 4.78 - 4.73 (m, 1H), 4.71 (d, J = 11.7 Hz, 1H), 3.83 (s, 3H), 3.83 - 3.75 (m, 1H), 3.58 - 3.54 (m, 1H), 1.79 - 1.52 (m, 6H)

### (ii) 1,1,1,3,3,3-Hexafluoro-2-(2-hydroxymethyl-4-methoxyphenyl)propan-2-ol

According to the procedure of the preparation of 2,2,2-trifluoro-1-(2-hydroxymethyl-4-methoxyphenyl)ethanol in Example 1, 1,1,1 ,3,3,3-hexafluoro-2-(4-methoxy-2-(tetrahydropyran-2-yloxymethyl)phenyl)propan-2-ol (350 mg, 0.90 mmol) was converted to the title compound (354 mg). This compound was used without further purification.

### (iii) 5-Methoxy-1,1-bistrifluoromethyl-1,3-dihydroisobenzofuran

A mixture of 1,1,1 ,3,3,3-hexafluoro-2-(2-hydroxymethyl-4-methoxyphenyl)propan-2-ol (300 mg) and conc. hydrochloric acid (3 ml) was stirred at 120°C for 6 hr. After cooling, the reaction mixture was diluted with water, extracted with dichloromethane. The organic layer was dried over magnesium sulfate and concentrated to give crude material of the title compound (258 mg). This was used without further purification.

### (iv) 6-Methoxy-3,3-bis(trifluoromethyl)-1,3-dihydroisobenzofuran-5-carbaldehyde

According to the procedure of the preparation of 6-methoxy-3-trifluoromethyl-1,3-dihydroisobenzofuran-5-carbaldehyde in Example 1, 5-methoxy-1,1-bistrifluoromethyl-1,3-dihydroisobenzofuran (258 mg) was converted to the title compound (167 mg, 69.0% from 1,1,1,3,3,3-hexafluoro-2-(2-hydroxymethyl-4-methoxyphenyl)propan-2-ol).
¹H-NMR (CDCl₃) : 10.44 (s, 1H), 7.98 (s, 1H), 6.98 (s, 1H), 5.36 (s, 2H), 4.00 (s, 3H)

### (v) 1-tert-Butoxycarbonyl-(2S,3S)-3-(6-methoxy-3,3-bis(trifluoromethyl)-1,3-dihydroisobenzofuran-5-yl)methylamino-2-phenylpiperidine

According to the procedure of the preparation of 1-tert-butoxycarbonyl-(2S,3S)-3-(6-methoxy-3-trifluoromethyl-1,3-dihydroisobenzofuran-5-yl)methylamino-2-phenylpiperidine in Example 1, 6-methoxy-3,3-bis(trifluoromethyl)-1,3-dihydroisobenzofuran-5-carbaldehyde (191 mg, 0.61 mmol) was converted to the title compound (327 mg, 93.3%).
¹H-NMR(CDCl₃) : 7.57 - 7.54 (m, 2H), 7.39 (s, 1H), 7.35 - 7.23 (m, 3H), 6.71 (s, 1H), 5.43 (m, 1H), 5.29 (s, 2H), 3.99 - 3.95 (m, 1H), 3.84 (s, 2H), 3.75 (s, 3H), 3.07 - 2.99 (m, 2H), 1.87 -1.33 (m, 5H), 1.39 (s, 9H)

### (vi) (2S,3S)-3-(6-Methoxy-3,3-bis(trifluoromethyl)-1,3-dihydroisobenzofuran-5-yl)methylamino-2-phenylpiperidine dihydrochloride

According to the procedure of the preparation of (2S,3S)-3-(6-methoxy-3-trifluoromethyl-1,3-dihydroisobenzofuran-5-yl)methylamino-2-phenylpiperidine dihydrochloride in Example 1, 1 -tert-butoxycarbonyl-(2S,3S)-3-(6-methoxy-3,3-bis(trifluoromethyl)-1,3-dihydroisobenzofuran-5-yl)methylamino-2-phenylpiperidine (327 mg, 0.57 mmol) was converted to the title compound (226 mg, 72.4%).
mp: 180-187°C
¹H-NMR(free amine, CDCl₃): 7.28 - 7.20(m, 5H), 7.16 (s, 1H), 6.56 (s, 1H), 5.27 (s, 2H), 3.89 (d, J = 2.6 Hz, 1H), 3.69 (d, J = 13.9 Hz, 1H), 3.52 (s, 3H), 3.35 (d, J = 13.9 Hz, 1H), 3.28 - 3.25 (m, 1H), 2.85 - 2.75 (m, 2H), 2.17 - 2.11 (m, 1H), 2.04 - 1.85 (m, 1H), 1.68 - 1.57 (m, 1H), 1.46 - 1.40 (m, 1H)

### Example 3

### Preparation of (2S.3S)-3-(6-Methoxy-1-methyl-1-trifluoromethylisochroman-7-yl)methylamino-2-phenylpiperidine dihydrochloride

### (i) 2-(2-Bromo-5-methoxyphenyl)ethanol

To a stirred mixture of 3-methoxyphenethylalcohol (1.18 g, 7.8 mmol) and pyridine (0.75 ml, 9.3 mmol) in dry dichloromethane (10 ml) was added bromine (0.47 ml, 18.0 mmol) dropwise under nitrogen at 0°C. The orange solution was stirred at room temperature for 4 hr. The reaction mixture was quenched by the addition of 10% sodium bisulfite aqueous solution., and extracted with dichloromethane. The organic extracts were washed with brine, dried over magnesium sulfate, and concentrated to give crude products, which were purified by silica-gel column chromatography eluted with gradient of hexane and ethyl acetate (10 : 1, 8 : 1, 5 : 1) to give the title compound as a colorless oil (1.5 g, 83.2%).
¹H-NMR (CDCl₃): 7.43 (d, J = 8.8 Hz, 1H), 6.83 (d, J = 3.3 Hz, 1H), 6.67 (dd, J = 8.8, 3.3 Hz, 1H), 3.91 - 3.81 (m, 2H), 3.78 (s, 3H), 2.99 (t, J = 6.6 Hz, 2H)

### (ii) 2-(2-(2-Bromo-5-methoxyphenyl)ethoxy)tetrahydropyran

According to the procedure of the preparation of 2-(2-bromo-5-methoxybenzyloxy)tetrahydropyran in Example 1, 2-(2-bromo-5-methoxyphenyl)ethanol (1.5 g, 6.5 mmol) was converted to the title compound (2.05 g, quant.).
¹H-NMR (CDCl₃): 7.40 (d, J = 8.8 Hz, 1H), 6.86 (d, J = 2.9 Hz, 1H), 6.65 (dd, J = 8.8, 2.9 Hz, 1H), 4.63 - 4.60 (m, 1H), 3.99 - 3.90 (m, 1H), 3.82 - 3.74 (m, 1H), 3.78 (s, 3H), 3.68 - 3.59 (m, 1H), 3.50 - 3.45 (m, 1H), 3.02 (t, J = 7.0 Hz, 2H), 1.83 - 1.52 (m, 6H)

### (iii) 1,1,1-Trifluoro-2-(4-methoxy-2-(2-(tetrahydropyran-2-yloxy)ethyl)phenyl)-propan-2-ol

To a stirred solution of 2-(2-(2-bromo-5-methoxyphenyl)-ethoxy)tetrahydropyran (1.0 g, 3.17 mmol) in dry tetrahydrofuran (20 ml) was added n-butyllithium (2.5 ml, 4.12 mmol) dropwise under nitrogen at -78°C. The reaction mixture was stirred at -40°C for 1 hr. To the reaction mixture was added a suspension of anhydrous cerium chloride (884 mg, 3.58 mmol) in dry tetrahydrofuran (15 ml) dropwise at -78°C and stirred for 1 hr. To the reaction mixture was added trifluoroacetone (0.5 ml, 5.59 mmol), and the resulting mixture was stirred at -78°C for 1 hr. This was quenched by saturated ammonium chloride solution, extracted with dichloromethane. The combined organic extracts were dried over magnesium sulfate, and concentrated to give a crude products, which were purified by silica-gel column chromatography eluted with a gradient of hexane and ethyl acetate (20 : 1, 15 : 1, 12 : 1, 10 : 1) to give the title compound (555 mg, 50.3%).
¹H-NMR(CDCl₃) : 7.35 - 7.31 (m, 1H), 6.78 - 6.74 (m, 2H), 5.70 and 5.62 (each s, total 1H), 4.63 and 4.48 (each m, total 1H), 4.18 - 4.11 and 3.99 - 3.92 (each m, total 1H), 3.80 (s, 3H), 3.77 - 3.43 (m, 3H), 3.33 - 2.90 (m, 2H), 1.80 and 1.78 (each s, total 3H), 1.75 - 1.26 (m, 6H)

### (iv) 6-Methoxy-1-methyl-1-trifluoromethylisochroman

A mixture of 1,1,1 -Trifluoro-2-(4-methoxy-2-(2-(tetrahydropyran-2-yloxy)ethyl)phenyl)-propan-2-ol (470 mg, 1.35 mmol) and conc. hydrochloric acid (4 ml) was stirred at 120°C for 3 hr. After cooling, the reaction mixture was diluted with water, and the aqueous layer was extracted with dichloromethane. The organic extracts were dried over magnesium sulfate, and concentrated to give the title compound as a brown oil (460 mg). This was used without further purification.

### (v) 6-Methoxy-1-methyl-1-trifluoromethylisochroman-7-carbaldehyde

According to the procedure of the preparation of 6-methoxy-3-trifluoromethyl-1,3-dihydroisobenzofuran-5-carbaldehyde in Example 1, 6-methoxy-1-methyl-1-trifluoromethylisochroman (460 mg) was converted to the title compound (179 mg, 48.3% from 1,1,1-Trifluoro-2-(4-methoxy-2-(2-(tetrahydropyran-2-yloxy)ethyl)phenyl)-propan-2-ol).
¹H-NMR(CDCl₃) : 10.41 (s, 1H), 7.82 (s, 1H), 6.78 (s, 1H), 4.19 - 4.11 (m, 1H), 3.94 (s, 3H), 3.94 - 3.87 (m, 1H), 2.91 (t, J = 4.4 Hz, 2H), 1.67 (s, 3H)

### (vi) 1-tert-Butoxycarbonyl-(2S,3S)-3-(6-methoxy-1-methyl-1-trifluoromethylisochroman-7-yl)methylamino-2-phenylpiperidine

According to the procedure of the preparation of 1-tert-butoxycarbonyl-(2S,3S)-3-(6-methoxy-3-trifluoromethyl-1,3-dihydroisobenzofuran-5-yl)methylamino-2-phenylpiperidine in Example 1, 6-methoxy-1-methyl-1-trifluoromethylisochroman-7-carbaldehyde (184 mg, 0.67 mmol) was converted to the title compound (330 mg, 91.8%).
¹H-NMR(CDCl₃) : 7.59 - 7.55 (m, 2H), 7.34 - 7.17 (m, 4H), 6.56 (s, 1H), 5.44 (m, 1H), 4.16 - 4.08 (m, 1H), 3.99 - 3.84 (m, 2H), 3.80 (m, 2H), 3.72 and 3.71 (each s, total 3H), 3.06 - 2.98 (m, 2H), 2.83 - 2.81 (m, 2H), 1.85 - 1.61 (m, 4H), 1.63 and 1.61 (each s, total 3H), 1 50 - 1.40 (m, 1H), 1.39 (s, 9H)

### (vii) (2S,3S)-3-(6-Methoxy-1-methyl-1-trifluoromethylisochroman-7-yl)methylamino-2-phenylpiperidine dihydrochloride

According to the procedure of the preparation of (2S,3S)-3-(6-methoxy-3-trifluoromethyl-1,3-dihydroisobenzofuran-5-yl)methylamino-2-phenylpiperidine dihydrochloride in Example 1, 1-tert-butoxycarbonyl-(2S,3S)-3-(6-methoxy-1-methyl-1-trifluoromethylisochroman-7-yl)methylamino-2-phenylpiperidine (325 mg, 0.61 mmol) was converted to the title compound (88 mg, 28.4%).
mp : 193-201°C
¹H-NMR (major isomer, free amine, CDCl₃) : 7.33 - 7.20 (m, 5H), 6.95 (s, 1H), 6.43 (s, 1H), 4.13 - 4.09 (m, 1H), 3.92 - 3.84 (m, 2H), 3.62 (d, J = 13.9 Hz, 1H), 3.51 (s, 3H), 3.33 (d, J = 13.9 Hz, 1H), 3.31 - 3.24 (m, 1H), 2.84 - 2.74 (m, 4H), 2.12 - 2.07 (m, 1H), 1.94 - 1.82 (m, 1H), 1.67 - 1.62 (m, 1H), 1.59 (s, 3H), 1.43 - 1.38 (m, 1H)
The diastereomeric ratio of epimers at the 1-position on the isochroman ring was determined by ¹H-NMR as 5 : 1 (1R:1S). These isomers are (2S,3S)-3-[(1R)- 6-methoxy-1-methyl- -trifluoromethylisochroman-7-yl]methylamino-2-phenylpiperidine and (2S,3S)-3-[(1S)-6-methoxy-1-methyl-1-trifluoromethylisochroman-7-yl]methylamino-2-phenylpiperidine.
More soluble epimer was recovered from the mother liquor. The diastereomeric ratio of epimers at the 1-position on the isochroman ring was determined by ¹H-NMR as 1 : 3 (1R:1S).
The absolute stereochemistry of the title compounds were determined by X-ray crystallography of the (3R) isomer after further purification by recrystallization.
¹H-NMR (major isomer, free amine, CDCl₃): 7.33-7.20 (m, 5H), 6.99 (s, 1H), 6.40 (s, 1H), 4.13 - 4.09 (m, 1H), 3.92 - 3.84 (m, 2H), 3.62 (d, J = 13.9 Hz, 1H), 3.45 (s, 3H), 3.33 (d, J = 13.9 Hz, 1H), 3.31 - 3.24 (m, 1H), 2.84 - 2.74 (m, 4H), 2.12 - 2.07 (m, 1H), 1.94 - 1.82 (m, 1H), 1.67 - 1.62 (m, 1H), 1.59 (s, 3H), 1.43 - 1.38 (m, 1H)

### Example 4

### Preparation of (2S,3S)-3-(6-Methoxy-3-methyl-3-trifluoromethyl-1,3-dihydroisobenzofuran-5-yl)methylamino-2-phenylpiperidine

### (i) 2-Bromo-5-methoxybenzylchloride

To a stirred solution of 3-methoxybenzylchloride (37.2 g, 0.238 mol) and pyridine (23.1 ml, 0.286 mol) dissolved in dry dichloromethane (400 ml) was added bromine (23 ml, 0.880 mol) at 0°C. The resulting mixture was stirred at 0°C for 1 hr, then at room temperature for 18 hr. This was diluted with a saturated aqueous solution of sodium thiosulfate, and extracted with dichloromethane. The combined extracts were washed with a saturated aqueous solution of sodium thiosulfate, water, 2N HCI, water and brine, sequentially. The extracts were dried over magnesium sulfate, and concentrated to give a crude product as a slight yellow crystal. This was dissolved with ethyl acetate, and the precipitate was filtered. The filtrate was washed, and concentrated to give a slightly yellow crystal which was washed with hexane to afford the title compound (43 g, 77%) as a white crystal.
¹H-NMR (CDCl₃) : 7.44 (d, J = 8.8 Hz, 1H), 7.02 (d, J = 3.3 Hz, 1H), 6.74 (dd, J = 8.8, 3.3 Hz, 1H), 4.64 (s, 2H), 3.79 (s, 3H)

### (ii) 5-Methoxy-1-methyl-1-trifluoromethyl-1,3-dihydroisobenzofuran

To a stirred solution of 2-bromo-5-methoxybenzylchloride (13.8 g, 0.059 mol) in a mixture of dry tetrahydrofuran (330 ml) and hexane (110 ml) was added n-butyllithium (37.2 ml, 0.062 mol) in hexane dropwise over 30 min at -100°C under nitrogen, and the reaction mixture was stirred at -100°C for 2.5 hr. Then, to the mixture was added a solution of 1,1,1-trifluoroacetone (6.3 ml, 0.071 mol) dissolved in dry tetrahydrofuran (15 ml) and hexane (5 ml) dropwise at the same temperature, and the resulting mixture was allowed to elevate to -30°C. This was quenched by water, and the solvent was removed by evaporation. The residue was extracted with hexane. The organic extracts were dried over magnesium sulfate, and concentrated to give crude products as a slight yellow oil (13.6 g). The crude oil (13.6 g), glycine (575 mg, 7.66 mmol) and potassium hydroxide (703 mg, 12.53 mmol) were dissolved in a mixture of ethanol (30 ml) and water (20 ml), and stirred at reflux for 2 hr. After cooling, the reaction mixture was diluted with brine, and extracted with hexane. The organic extracts were dried over magnesium sulfate, and concentrated by evaporation to afford a slight yellow oil (12.6 g), which was purified by distillation (94 to 98°C/1.5 mmHg) to give the title compound as a colorless oil (10.8 g, 78.7%).
¹H-NMR(CDCl₃): 7.20 (d, J = 8.4 Hz, 1H), 6.87 (dd, J = 8.4, 2.6 Hz, 1H), 6.76 (d, J = 2.6 Hz, 1H), 5.21 - 5.09 (m, 2H), 3.82 (s, 3H), 1.65 (d, J = 1.1 Hz, 3H)

### (iii) 3-Methyl-3-trifluoromethyl-6-methoxy-1,3-dihydroisobenzofuran-5-carbaldehyde

To a stirred solution of 5-methoxy-1-methyl-1-trifluoromethyl-1,3-dihydroisobenzofuran (10.8 g, 0.046 mol) in dry dichloromethane (280 ml) was added titanium (IV) chloride (11.2 ml, 0.102 mol) dropwise under nitrogen at -78°C, and the resulting solution was stirred for 15 min. A solution of dichloromethyl methyl ether (8.4 ml, 0.093 mol) in dry dichloromethane (20 ml) was added to the resulting brown solution at -78°C, and stirred for 1.5 hr. This mixture was poured onto ice-water, and stirred at room temperature for 30 min. The organic layer was separated, and the aqueous layer was extracted with dichloromethane. The combined organic extracts were washed with brine, dried over magnesium sulfate, and concentrated to afford the title compound as a slight yellow crystal (12.1 g, quant.).
¹H-NMR(CDCl₃) : 10.45 (s, 1H), 7.79 (s, 1H), 6.88 (s, 1H), 5.25 - 5.13 (m, 2H), 3.97 (s, 3H), 1.68 (m, 3H)

### (iv) (2S,3S)-3-(6-Methoxv-3-methvl-3-trifluoromethyl-1,3-dihydroisobenzofuran-5-yl)methylamino-2-phenylpiperidine

To a stirred solution of (2S,3S)-2-phenyl-3-aminopiperidine (4.1 g, 23.1 mmol) which was prepared by a method described in International Patent Publication WO 92/17449, and 3-methyl-3-trifluoromethyl-6-methoxy-1,3-dihydroisobenzofuran-5-carbaldehyde (6.1 g, 23.3 mol) in dry dichloromethane (200 ml) was added sodium triacetoxyborohydride (7.8 g, 36.9 mmol) portionwise under nitrogen at room temperature, and the resultant mixture was stirred at the same temperature for 16 hr. The pH was adjusted to below 10 with a saturated aqueous sodium bicarbonate solution, and extracted with dichloromethane. The extracts were dried over magnesium sulfate, and concentrated to afford a slight yellow amorphous solid (10.1 g). A methanolic HCl solution was added to the crude products dissolved in ethyl acetate. Formed solids were collected by filtration, dried under vacuum, and then purified by crystallization from methanol to afford the title compound as a white crystal.
mp: 200-207°C
¹H-NMR(major isomer, free amine, CDCl₃): 7.31 - 7.21 (m, 5H), 6.89 (s, 1H), 6.54 (s, 1H), 5.16 - 5.04 (m, 2H), 3.90 (d, J = 2.3 Hz, 1H), 3.68 (d, J = 14.3 Hz, 1H), 3.52 (s, 3H), 3.40 (d, J = 14.3 Hz, 1H), 3.29 - 3.26 (m, 1H), 2.85 - 2.75 (m, 2H), 2.14 - 2.09 (m, 1H), 1.95 - 1.76 (m, 1H), 1.66 - 1.54 (m, 1H), 1.60 (s, 3H), 1.44 - 1.40 (m, 1H)
Analysis by ¹H NMR indicated the diastereomeric ratio at the 3-position of the dihydroisobenzofuran ring as 98:2 (3R:3S). These isomers are (2S,3S)-3-[(3R)-6-Methoxy-3-methyl-3-trifluoromethyl-1,3-dihydroisobenzofuran-5-yl]methylamino-2-phenylpiperidine and (2S,3S)-3-[(3S)-6-Methoxy-3-methyl-3-trifluoromethyl-1,3-dihydroisobenzofuran-5-yl]methylamino-2-phenylpiperidine.
From the residual mother liquor; the other epimer at the 3-position on the dihydroisobenzofuran ring was recovered as a 9:1 (35:3R) mixture.
The absolute stereochemistry of the title compounds was determined by X-ray crystallography of the (3R) isomer after further purification by recrystallization.
1H-NMR (major isomer, free amine, CDCl₃) : 7.31 - 7.19 (m, 5H), 6.94 (s, 1H), 6.51 (s, 1H), 5.16 - 5.04( m, 2H), 3.89 (d, J = 2.2 Hz, 1H), 3.67 (d, J = 14.3 Hz, 1H), 3.48 (s, 3H), 3.37 (d, J = 14.3 Hz, 1H), 3.28 - 3.24 (m, 1H), 2.85 - 2.75 (m, 2H), 2.14 - 2.09 (m, 1H), 1.97 - 1.86 (m, 1H), 1.69 - 1.56 (m, 1H), 1.59 (s, 3H), 1.45 - 1 40 (m, 1H)

### Example 5

### Preparation of (2S,3S)-3-(6-Methoxy-3-phenyl-3-trifluoromethyl-1,3-dihydroisobenzofuran-5-yl)methylamino-2-phenylpiperidine dihydrochloride

### (i) 1-Trifluoromethyl-5-methoxy-1-phenyl-1,3-dihydroisobenzofuran

To a stirred solution of 2-bromo-5-methoxybenzylchloride (3.0 g, 12.7 mmol) in a mixture of dry tetrahydrofuran (60 ml) and hexane (20 ml) was added n-butyllithium (8.4 ml, 13.4 mmol) in hexane dropwise over 15 min at -85°C under nitrogen, and the reaction mixture was stirred at -85°C for 2 hr. Then, to the mixture was added a solution of 2,2,2-trifluoroacetophenone (2.70g, 15.3mmol) dissolved in dry tetrahydrofuran (20 ml) dropwise at the same temperature, and the resulting mixture was allowed to elevate to room temperature. This was quenched by water, and the solvent was removed by evaporation. The residue was extracted with dichloromethane. The organic extracts were dried over magnesium sulfate, and concentrated to give crude products as a dark yellow oil. The crude oil was purified by the method using glycine described for the synthesis of 5-methoxy-1-methyl-1-trifluoromethyl-1,3-dihydroisobenzofuran in Example 4 and by column chromatography on silica gel (20 g) with hexane-ethyl acetate (20 : 1) to give the title compound as a slight yellow oil (760 mg, 20.3 %).
¹H-NMR(CDCl₃) : 7.74 - 7.66 (m, 2H), 7.52 - 7.28 (m, 4H), 6.90 (dd, J = 8.6, 2.5 Hz, 1H), 6.80 - 6.76 (m, 1H), 5.33 (d, J = 12.2 Hz, 1H), 5.23 (d, J = 12.2 Hz, 1H), 3.82 (s, 3H).

### (ii) 3-Trifluoromethyl-6-methoxy-3-phenyl-1,3-dihydroisobenzofuran-5-carbaldehyde

According to the procedure of the preparation of 6-methoxy-3-trifluoromethyl-1,3-dihydroisobenzofuran-5-carbaldehyde in Example 1, 1-trifluoromethyl-5-methoxy-1-phenyl-1,3-dihydroisobenzofuran was converted, and the crude product was purified by column chromatography on silica gel (70 g) with hexane-ethyl acetate (5 : 1) to give the title compound as a yellow viscous oil (507 mg,61.7%).
¹H-NMR(CDCl₃) : 10.45 (s, 1H), 8.06 (s, 1H), 7.75 - 7.66 (m, 2H), 7.44 - 7.30 (m, 3H), 6.90 (s, 1H), 5.38 (d, J = 13.4 Hz, 1H), 5.27 (d, J = 13.4 Hz, 1H), 3.96 (s, 3H).

### (iii) 1-tert-Butoxycarbonyl-(2S,3S)-3-(6-Methoxy-3-phenyl-3-trifluoromethyl-1,3-dihydroisobenzofuran-5-yl)methylamino-2-phenylpiperidine

According to the procedure of the preparation of 1-tert-butoxycarbonyl-(2S,3 S)-3-(6-methoxy-3-trifluoromethyl-1,3-dihydroisobenzofuran-5-yl)methylamino-2-phenylpiperidine in Example 1, 3-trifluoromethyl-6-methoxy-3-phenyl-1,3-dihydroisobenzofuran-5-carbaldehyde (453 mg, 1.41 mmol) was converted, and the crude product was purified by column chromatography on silica gel (40 g) with dichloromethane-methanol (80 : 1) to give the title compound (657 mg) as a pale yellow oil. This was employed for the next step without further purification.

### (iv) (2S,3S)-3-(6-Methoxy-3-phenyl-3-trifluoromethyl-1,3-dihydroisobenzofuran-5-yl)methylamino-2-phenylpiperidine.

To a stirred solution of 1 -tert-butoxycarbonyl-(2S,3S)-3-(6-methoxy-3-phenyl-3-trifluoromethyl-1,3-dihydroisobenzofuran-5-yl)methylamino-2-phenylpiperidine (657 mg) in ethyl acetate (25 ml) was added conc. hydrochloric acid (3 ml) with ice-cooling. The reaction mixture was stirred at room temperature for 1.5 hr. The pH of the mixture was adjusted to below 10 with 2N sodium hydroxide with ice-cooling. The organic layer was separated, and the aqueous layer was extracted with ethyl acetate. The combined extracts were washed with brine, dried over magnesium sulfate, and concentrated to give crude products (559 mg) as a yellow oil. The crude products were purified by column chromatography on silica gel (18 g) with dichloromethane-methanol (40 : 1 to 10 : 1) to give the title compound as a yellow viscous oil (497 mg, 87.9 %).

### (v) (2S,3S)-3-(6-Methoxy-3-phenyl-3-trifluoromethyl-1,3-dihydroisobenzofuran-5-yl)methylamino-2-phenylpiperidine dihydrochloride

(2S,3S)-3-(3-trifluoromethyl-6-methoxy-3-phenyl-1,3-dihydroisobenzofuran-5-yl)methylamino-2-phenylpiperidine (497 mg, 1.03 mmol) was treated with 10% hydrogen chloride-methanol solution (20 ml). After the solvent was removed under vacuum, the residue was washed with hot ethanol to give the title compound as a white solid.
mp. 203-204 °C

The diastereomeric ratio of the epimers at 3-position on the dihydroisobenzofuran ring was determined by ¹H-NMR as 6.5: 1
¹H-NMR(major isomer, free amine, CDCl₃) : 7.71 - 7.62 (m, 2H), 7.45 - 7.17 (m, 9H), 6.55 (s, 1H), 5.29 (d, J = 12.2 Hz, 1H), 5.19 (d, J = 12.2 Hz, 1H), 3.88 (d, J = 2.1 Hz, 1H), 3.67 (d, J =14.3 Hz, 1H), 3.51 (s, 3H), 3.42 (d, J =14.3 Hz, 1H), 3.35 - 3.23 (m, 1H), 2.89 - 2.73 (m, 2H), 2.20 - 1.78 (m, 4H), 1.70 - 1.35 (m, 2H).

More soluble epimer was recovered from the mother liquor as a 2 : 1 mixture.
¹H-NMR(major isomer, free amine, CDCl₃) : 7.70 - 7.60 (m, 2H), 7.45 - 7.15 (m, 9H), 6.52 (s, 1H), 5.29 (d, J = 12.2 Hz, 1H), 5.19 (d, J = 12.2 Hz, 1H), 3.90 (d, J = 2.5 Hz, 1H), 3.72 (d, J =14.0 Hz, 1H), 3.47 (s, 3H), 3.33 (d, J =14.0 Hz, 1H), 3.33 - 3.21 (m, 1H), 2.88 - 2.72 (m, 2H), 2.18-1.78 (m, 4H), 1.72 - 1.35 (m, 2H).

### Example 6

### Preparation of (2S,3S)-3-[1-(6-Methoxy-3-methyl-3-trifluoromethyl-1,3-dihydroisobenzofuran-5-yl)ethylamino]-2-phenylpiperidine dihydrochloride

### (i) 5-Acetyl-3-methyl-6-methoxy-3-trifluoromethyl-1,3-dihydroisobenzofuran

To a solution of aluminum chloride (689 mg, 5.17 mmol) in dry dichloromethane (10 ml) was added acetyl chloride (0.37 ml, 5.17 mmol) at 0°C, and stirred for 10 min. A solution of 5-methoxy-1-methyl-1-trifluoromethyl-1,3-dihydroisobenzofuran (1.00 g, 4.31 mmol) in dry dichloromethane (10 ml) was added dropwise to the mixture at 0°C, and the resulting solution was stirred at the same temperature for 1 hr. This mixture was poured onto a mixture of ice-hydrochloric acid (1 N), and the organic layer was separated. The aqueous layer was extracted with dichloromethane, and the organic fractions were combined. The organic extracts were washed with brine, dried over sodium sulfate, and concentrated under reduced pressure. The residue was purified by column chromatography on silica gel with hexane and ethyl acetate (8 : 1) to give the title compound as a white crystal (1.10 g, 93%).
¹H-NMR (CDCl₃) : 7.69 (s, 1H), 6.86 (s, 1H), 5.21(d, J = 13.2 Hz, 1H), 5.14 (d, J = 13.2 Hz, 1H), 3.94 (s, 3H), 2.62 (s, 3H), 1.67 (s, 3H)

### (ii) (2S,3S)-3-[1-(6-Methoxy-3-methyl-3-trifluoromethyl-1.3-dihydroisobenzofuran-5-yl)ethylamino]-2-phenylpiperidine

To a stirred mixture of (2S,3S)-2-phenyl-3-aminopiperidine (350 mg, 1.99 mmol), 5-acetyl-3-methyl-6-methoxy-3-trifluoromethyl-1,3-dihydroisobenzofuran (545 mg, 1.99 mmol) and triethylamine (0.83 ml, 5.96 mmol) in dry dichloromethane (20 ml) was added titanium tetrachloride (0.11 ml, 0.99 mmol) dropwise at 0°C. The mixture was stirred at room temperature for 1 hr, and then cooled to at 0°C. Sodium cyanoborohydride (374 mg, 5.96 mmol) in methanol (5 ml) was added at the same temperature, and the mixture was allowed to warm to room temperature and stirred for 30 min. Hydrochloric acid (1 N, 15 ml) was added, and the mixture was stirred at room temperature for 1 hr. Ethyl acetate (80 ml) was added to the resultant mixture, and the mixture was extracted with hydrochloric acid (1 N, 60 ml x 3). The combined aqueous extracts were washed with ethyl acetate (60 ml x 2), and the pH was adjusted to pH9 with saturated aqueous potassium carbonate. The aqueous layer was extracted with ethyl acetate (60 ml x 3), and the combined organic fractions were washed with saturated sodium bicarbonate solution (60 ml). The organic solution was dried over sodium sulfate, and evaporated under vacuum to give a crude product. This was purified by silica-gel column chromatography eluted with hexane : ethylacetate (10 : 1) to afford the title compounds as a colorless foam (145 mg, 17 %).
¹H-NMR analysis showed this to consist of a 5 : 5 : 2 : 2 mixture of four diastereomers based on C-3 of the dihydroisobenzofuran and C1 of the ethylamino part.
¹H-NMR (C₆D₆, partial data) : 1.70, 1.59, 1.56 and 1.52 (four singlets, total 3H. The ratio was 5 : 5 : 2 : 2 respectively.)

### (iii) (2S,3S)-3-[1-(6-Methoxy-3-methyl-3-trifluoromethyl-1,3-dihydroisobenzofuran-5-yl)ethylamino]-2-phenylpiperidine dihydrochloride

To a stirred solution of (2S,3S)-3-[1-(6-methoxy-3-methyl-3-trifluoromethyl-1,3-dihydroisobenzofuran-5-yl)ethylamino]-2-phenylpiperidine (106 mg, 0.24 mmol) in methanol (1 ml) was added 10 % methanolic hydrogen chloride (10 ml), followed by stirring at room temperature for 30 min. The mixture was concentrated under vacuum, and the residue was crystallized from methanol and diethyl ether to give a white solid (45 mg, 36 %).

The diastereomeric ratio of this solid was determined by ¹H-NMR analysis as 20 : 30 : 1 : 3.
¹H-NMR (free base, C₆D₆, partial data) : 6.06 (s, 1H), 5.00 (d, J = 12.0 Hz, 1H), 4.83 (d, J = 12.0 Hz, 1H), 3.59 - 3.36 (m, 2H), 3.18, 3.17 and 3.13 (3s, total 3H), 3.07 - 3.01 (m, 1H), 2.68 - 2.63 (m, 1H), 2.49 - 2.37 (m, 1H), 1.73, 1.62, 1.56 and 1.53 (four singlets, total 3H. The ratio was 20 : 30 : 1 : 3 respectively.), 1.31, 1.28, 1.07 and 1.01 (four doublets, J = 5.6, 5.6, 6.3 and 6.6 Hz respectively, total 3H)

The mother liquor was evaporated under reduced pressure. The residual solid was washed with diethyl ether, and dried to afford a slight yellow solid (39 mg, 32 %).
¹H-NMR analysis of this solid showed the diastereomeric ratio as 5 : 1 : 5 : 4.
¹H-NMR(free base, C₆D₆, partial data) : 6.06 (s, 1H), 5.03 - 4.96 (m, 1H), 4.85 - 4.77 (m, 1H), 3.18, 3.17 and 3.13 (3s, total 3H), 1.71, 1.58, 1.53 and 1.52 (four singlets, total 3H. The ratio was 5 : 1 : 5 : 4 respectively.), 1.29, 1.26, 1.05 and 1.00 (four doublets, J = 4.5, 4.5, 6.3 and 6.6 Hz respectively, total 3H)

### Example 7

### Preparation of (2S,3S)-3-[(1R)-6-Methoxy-1-methyl-1-trifluoromethylisochroman-7-yl]methylamino-2-phenylpiperidine dihydrochloride

### (i) 6-Hydroxy-1-methyl-1-trifluoromethylisochroman

To a stirred solution of 6-Methoxy-1-methyl-1-trifluoromethylisochroman (71 g, 0.29 mol) in AcOH (600 mL) was added aqueous 48% HBr (300 mL) and the mixture was stirred at 130 °C for 13 hr. After removing AcOH *in vacuo*, the reaction mixture was treated with aqueous NaOH (8 M) until the pH became 5-6. The resultant solution was extracted with EtOAc (400 mL x 2) and the combined EtOAc extracts were washed with brine(100 mL), dried over MgSO₄, and concentrated *in vacuo*. Flash chromatography (Silica-gel, 15 x 20 cm, 17% AcOEt-hexane) afforded 6-hydroxy-1-methyl-1-trifluoromethylisochroman (67g, 100%) as a colorless oil.
¹H-NMR(CDCl₃) : 7.22 (d, J = 9.1 Hz, 1H), 6.73 (dd, J = 9.1, 2.6 Hz, 1H), 6.63 (d, J = 2.6 Hz, 1H), 5.00 (s, 1H), 4.17-4.07 (m, 1H), 3.90 (dt, J = 11, 5.8 Hz, 1H), 2.84-2.78 (m, 2H), 1.64 (s, 3H).

### (ii) 6-Acetoxy-1-methyl-1-trifluoromethylisochroman

To a stirred solution of 6-hydroxy-1-methyl-1-trifluoromethylisochroman (79g, 0.34 mol) and triethylamine (120 mL, 0.88 mol) in THF (680 mL) was added acetyl chloride (31 mL, 0.44 mol) at 0 °C, and the mixture was stirred at room temperature for 1 hr. The reaction was quenched by adding aqueous 1 N-HCl (400 mL), and extracted with AcOEt (500 mL). The extracts were washed with aqueous saturated NaHCO₃ (100 mL) and brine (100 mL), dried over MgSO₄, and concentrated *in vacuo*.. The residue was purified by flash chromatography (Silica-gel, 15 x 20 cm, 6% AcOEt-hexane) to afford 6-acetoxy-1-methyl-1-trifluoromethylisochroman (83g, 89%) as a colorless oil.
¹H-NMR(CDCl₃): 7.36 (d, J = 7.2 Hz, 1H), 6.98 (dd, J = 7.2, 2.5 Hz, 1H), 6.91 (d, J = 2.5 Hz, 1H), 4.18-4.08 (m, 1H), 3.92 (dt, J = 11, 5.4 Hz, 1H), 2.86 (t, J = 5.4 Hz, 2H), 2.30 (s, 1H), 1.66 (s, 3H).

### (iii)(1R)-6-Acetoxy-1-methyl-1-trifluoromethyl-isochroman and (1S)-6-Hydroxy-1-methyl-1-trifluoromethyl-isochroman

A mixture of racemic 6-acetoxy-1-methyl-1-trifluoromethylisochroman (38.4g, 0.140 mol), 10% sec-butanol solution in hexane (1.3 L), and lipase PS (35 g) was stirred vigorously at room temperature for 23 hr. After filtration, the filtrate was concentrated under reduced pressure to give a mixture. This was purified by silica-gel column chromatography eluted with gradient of hexane and ethyl acetate (15:1,5:1,2:1) to give, first, (lR)-6-acetoxy-1-methyl-1-trifluoromethyl-isochroman as a colorless oil (17.3g, 45%, 94%ee). The ¹H-NMR spectra of this compound was identical with that of racemate. The second fraction gave (1S)-6-hydroxy-1-methyl-1-trifluoromethyl-isochroman as crystals (16.9g, 52%, 83%ee). The ¹H-NMR spectra of this material was identical with that of racemate.

### (iv) (1R)-6-Hydroxy-1-methyl-1-trifluoromethyl-isochroman

To a stirred mixture of (lR)-6-acetoxy-1-methyl-trifluoromethylisochroman (35.5g, 0.129 mol), methanol (860 mL), and water (340) was added potassium carbonate (35.7g, 0.258 mol) at 0°C, then the mixture was stirred at room temperature for 1 hr. The resultant mixture was acidified with 2 N hydrochloric acid (pH 3) and evaporated in vacuo to remove methanol. The residue was extracted with ethyl acetate. The organic layer was washed with water and brine, and dried over magnesium sulfate. After filtration, the filtrate was concentrated under reduced pressure to afford the title compound as a colorless oil (28.0g, 93%). This was used without further purification. The ¹H-NMR spectra of this compound was identical with that of the racemate.

### (v) (1R)-6-Methoxy-1-methyl-1-trifluoromethyl-isochroman

To a stirred mixture of sodium hydride (3.47 g, 0.145 mol) in DMF (50 mL) was added (lR)-6-hydroxy-1-methyl-1-trifluoromethylisochroman (28.0 g, 0.121 mol) solution in DMF (370 mL) at 0°C, then the mixture was stirred at room temperature for 1 hr. The reaction mixture was quenched with water and diluted with saturated aqueous ammonium chloride. This was extracted with ethyl acetate-toluene (4 : 1). The organic fraction was washed with water and brine, and dried over magnesium sulfate. The solvent was removed in vacuo, the residue was purified by column chromatography on silica-gel eluted with hexane and ethyl acetate (40 : 1) to give the title compound as a colorless oil (29.1 g, 98%). The ¹H-NMR spectra of this material was identical with that of racemate.

### (vi)(2S,3S)-3-[(1R)-6-Methoxy-1-methyl-1-trifluoromethylisochoman-7-yl]methylamino-2-phenylpiperidine dihydrochloride

The above (1R)-6-Methoxy-1-methyl-1-trifluoromethyl-isochroman was further converted to the title compound by following the method for preparation of Example 3 to afford the title compound in a single diastereomeric form.
Optical Rotation: [α]²⁷_{D}= +75.44° (c=0.424, MeOH)

The chemical structures of the compounds of the formula (I) prepared in Examples 1 to 7 are summarized in the following table.

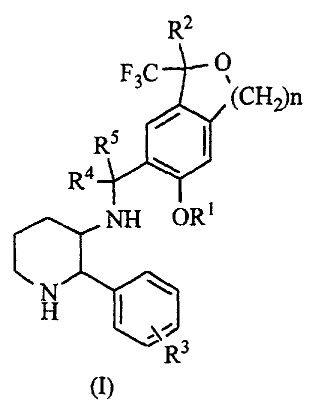

**TABLE**

| | | | | | | | |
|---|---|---|---|---|---|---|---|
| Example No. | R¹ | R² | R³ | R⁴ | R⁵ | n | RS-configuration of piperidine ring |
| 1 | CH₃ | H | H | H | H | 1 | (2*S*,3*S*) |
| 2 | CH₃ | CF₃ | H | H | H | 1 | (2*S*,3*S*) |
| 3 | CH₃ | CH₃ | H | H | H | 2 | (2*S*,3*S*) |
| 4 | CH₃ | CH₃ | H | H | H | 1 | (2*S*,3*S*) |
| 5 | CH₃ | C₆H₅ | H | H | H | 1 | (2*S*,3*S*) |
| 6 | CH₃ | CH₃ | H | CH₃ | H | 1 | (2*S*,3*S*) |
| 7 | CH₃ | CH₃ | H | H | H | 2 | (2*S*,3*S*) |

## Claims

1. A compound of the formula (I): 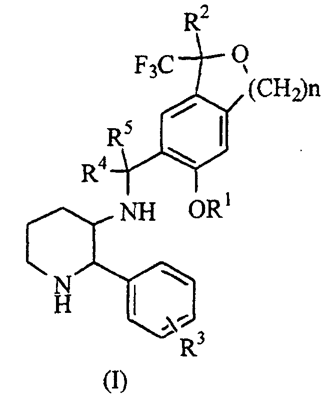 and its pharmaceutically acceptable salts, wherein
R¹ is C₁-C₆ alkyl;
R² is hydrogen, C₁-C₆ alkyl, halo C₁-C₆ alkyl or phenyl;
R³ is hydrogen or halo;
R⁴ and R⁵ are independently hydrogen, C₁-C₆ alkyl or halo C₁-C₆ alkyl;
and n is one, two or three.

2. A compound according to Claim 1, wherein
R¹ is C₁-C₃ alkyl;
R² is hydrogen, C₁-C₃ alkyl, halo C₁-C₃ alkyl or phenyl;
R³ is hydrogen or fluorine;
R⁴ and R⁵ are independently hydrogen, C₁-C₃ alkyl or halo C₁-C₃ alkyl;
and
n is one or two.

3. A compound according to Claim 2, wherein R¹ is methyl; R² is hydrogen, methyl, trifluoromethyl or phenyl; R³ is hydrogen; and R⁴ and R⁵ are hydrogen.

4. A compound according to Claim 3 selected from:
(2*S*,3*S*)-3-(6-methoxy-3-trifluoromethyl-1,3-dihydroisobenzofuran-5-yl)methylamino-2-phenylpiperidine or its salts;
(2*S*,3*S*)-3-(6-methoxy-1-methyl-1 -trifluoromethylisochroman-7-yl)methylamino-2-phenylpiperidine or its salts;
(2*S*,3*S*)-3-(6-methoxy-3-methyl-3-trifluoromethyl-1,3-dihydroisobenzofuran-5-yl)methylamino-2-phenylpiperidine or its salts;
(2*S*,3*S*)-3-(6-methoxy-3-phenyl-3-trifluoromethyl-1,3-dihydroisobenzofuran-5-yl)methylamino-2-phenylpiperidine or its salts; and
(2S,3S)-3-[1-(6-methoxy-3-methyl-3-trifluoromethyl-1,3-dihydroisobenzofuran-5-yl)ethylamino]-2-phenylpiperidine or its salts.

5. A compound according to Claim 4 wherein said compound is
(2S,3S)-3-[(1R)-6-Methoxy-1-methyl-1-trifluoromethylisochroman-7-yl]methylamino-2-phenylpiperidine or its salts; or
(2S,3S)-3-[(3R)-(6-Methoxy-3-methyl-3-trifluoromethyl-1,3-dihydroisobenzofuran-5-yl)]methylamino-2-phenylpiperidine or its salts.

6. A compound of the formula (III): 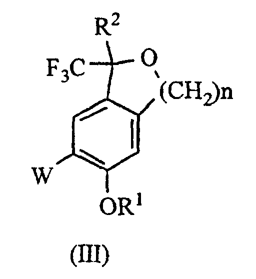 wherein W is hydrogen or Q(O=)C- wherein Q is H, C₁-C₆ alkyl or halo C₁-C₆ alkyl; R¹ is C₁-C₆ alkyl; R² is hydrogen, C₁-C₆ alkyl, halo C₁-C₆ alkyl or phenyl; and n is one, two or three.

7. A compound according to Claim 6 wherein said compound is selected from:
5-methoxy- -trifluoromethyl-1,3-dihydroisobenzofuran;
6-methoxy-3-trifluoromethyl-1,3-dihydrobenzofuran-5-carbaldehyde;
5-methoxy-1,1-bistrifluoromethyl-1,3 -dihydroisobenzofuran;
6-methoxy-3,3-bis(trifluoromethyl)-1,3-dihydroisobenzofuran-5-carbaldehyde;
6-methoxy-1-methyl-1-trifluoromethylisochroman;
6-methoxy- -methyl-1 -trifluoromethylisochroman-7-carbaldehyde;
5-methoxy-1 -methyl-1-trifluoromethyl-1,3-dihydroisobenzofuran;
6-methoxy-3-methyl-3-trifluoromethy-1,3-dihydroisobenzofuran-5-carbaldehyde;
1-trifluoromethyl-5-methoxy-1-phenyl-1,3-dihydroisobenzofuran;
3-trifluoromethyl-6-methoxy-3-phenyl-1,3-dihydroisobenzofuran-5-carbaldehyde;
5-acetyl-3-methyl-6-methoxy-3-trifluoromethyl-1,3-dihydroisobenzofuran;
(1R)-6-methoxy-1-methyl-1-trifluoromethylisochroman;
(1R)-6-methoxy-1-methyl-1-trifluoromethylisochroman-7-carbaldehyde;
(1S)-6-methoxy-1-methyl-1-trifluoromethylisochroman;
(1S)-6-methoxy-1-methyl-1-trifluoromethylisochroman-7-carbaldehyde;
(1R)-5-methoxy-1-methyl-1-trifluoromethyl-1,3-dihydroisobenzofuran;
(1R)-6-methoxy-3-methyl-3-trifluoromethyl-1,3-dihydroisobenzofuran-5-carbaldehyde;
(1S)-5-methoxy-1-methyl-1-trifluoromethyl-1,3-dihydroisobenzofuran; and
(1S)-6-methoxy-3-methyl-3-trifluoromethyl-1,3-dihydroisobenzofuran-5-carbaldehyde.

8. A pharmaceutical composition comprising a compound of formula (I), as defined in claim 1 or a pharmaceutically acceptable salt thereof, and a pharmaceutically acceptable carrier.

9. A composition according to Claim 8 suitable for treating a disorder or condition in a mammal for which a substance P antagonist is indicated.

10. A composition according to Claim 9 wherein said disorder or condition is selected from cardiovascular diseases, allergic disorders, angiogenesis, gastrointestinal disorders, central nervous system disorders, inflammatory diseases, emesis, urinary incontinence, pain, migraine, severe anxiety disorders, stress disorders, anxiety, major depressive disorders, major depressive disorders with anxiety, depression, sunburn, sexual dysfunction, bipolar disorders, substance use disorders, schizophrenic disorders, movement disorders, cognitive disorders, and diseases, disorders and adverse conditions caused by *Helicobacter pylori.*

11. A compound of formula (I) as defined in claim 1 for use as a medicament.

12. The use of a compound of formula (I) as defined in claim 1 for the preparation of a medicament for the treatment of a disorder or condition in a mammal for which a substance P antagonist is indicated.

13. Use according to Claim 12 wherein said disorder or condition is selected from cardiovascular diseases, allergic disorders, angiogenesis, gastrointestinal disorders , central nervous system disorders, inflammatory diseases, emesis, urinary incontinence, pain, migraine, severe anxiety disorders, stress disorders, anxiety, major depressive disorders, major depressive disorders with anxiety, depression, sunburn, sexual dysfunction, bipolar disorders, substance use disorders, schizophrenic disorders, movement disorders, cognitive disorders, and diseases, disorders and adverse conditions caused by *Helicobacter pylori.*

## Patentansprüche

1. Verbindung der Formel (I): 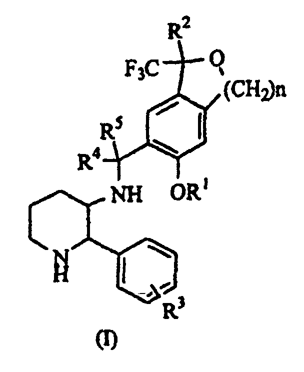 und ihre pharmazeutisch verträglichen Salze, wobei
R¹ ein C₁-C₆-Alkylrest ist;
R² ein Wasserstoffatom, ein C₁-C₆-Alkylrest, ein HalogenC₁-C₆-alkylrest oder eine Phenylgruppe ist;
R³ ein Wasserstoffatom oder ein Halogenatom ist;
R⁴ und R⁵ unabhängig voneinander Wasserstoffatome, C₁-C₆-Alkylreste oder HalogenC₁-C₆-alkylreste sind;
und
n eins, zwei oder drei ist.

2. Verbindung gemäß Anspruch 1, wobei
R¹ ein C₁-C₃-Alkylrest ist;
R² ein Wasserstoffatom, ein C₁-C₃-Alkylrest, ein HalogenC₁-C₃-Alkylrest oder eine Phenylgruppe ist;
R³ ein Wasserstoffatom oder ein Fluoratom ist;
R⁴ und R⁵ unabhängig voneinander Wasserstoffatome, C₁-C₃-Alkylreste oder HalogenC₁-C₃-alkylreste sind;
und
n eins oder zwei ist.

3. Verbindung gemäß Anspruch 2, wobei R¹ eine Methylgruppe ist; R² ein Wasserstoffatom, eine Methylgruppe, eine Trifluormethylgruppe oder eine Phenylgruppe ist; R³ ein Wasserstoffatom ist; und R⁴ und R⁵ Wasserstoffatome sind.

4. Verbindung gemäß Anspruch 3, ausgewählt aus:
(2S,3S)-3-(6-Methoxy-3-trifluormethyl-1,3-dihydroisobenzofuran-5-yl)methylamino-2-phenylpiperidin oder dessen Salze;
(2S,3S)-3-(6-Methoxy-1-methyl-1-trifluormethylisochroman-7-yl)methylamino-2-phenylpiperidin oder dessen Salze;
(2S,3S)-3-(6-Methoxy-3-methyl-3-trifluormethyl-1,3-dihydroisobenzofuran-5-yl)methylamino-2-phenylpiperidin oder dessen Salze;
(2S,3S)-3-(6-Methoxy-3-phenyl-3-trifluormethyl-1,3-dihydroisobenzofuran-5-yl)methylamino-2-phenylpiperidin oder dessen Salze; und
(2S,3 S)-3-[1-(6-Methoxy-3-methyl-3-trifluormethyl-1,3-dihydroisobenzofuran-5-yl)ethylamino]-2-phenylpiperidin oder dessen Salze.

5. Verbindung gemäß Anspruch 4, wobei die Verbindung
(2S,3S)-3-[(lR)-6-Methoxy-1-methyl-1-trifluormethylisochroman-7-yl]methylamino-2-phenylpiperidin oder dessen Salze; oder
(2S,3S)-3-[(3R)-(6-Methoxy-3-methyl-3-trifluormethyl-1,3-dihydroisobenzofuran-5-yl)]methylamino-2-phenylpiperidin oder dessen Salze ist.

6. Verbindung der Formel (III): 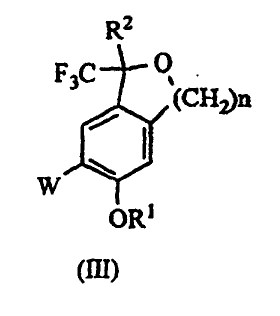 wobei W ein Wasserstoffatom oder Q(O=)C- ist, wobei Q ein Wasserstoffatom, ein C₁-C₆-Alkylrest oder HalogenC₁-C₆-alkylrest ist; R¹ ein C₁-C₆-Alkylrest ist; R² ein Wasserstoffatom, ein C₁-C₆-Alkylrest, ein HalogenC₁-C₆-alkylrest oder eine Phenylgruppe ist; und n eins, zwei oder drei ist.

7. Verbindung gemäß Anspruch 6, wobei die Verbindung ausgewählt ist aus:
5-Methoxy- 1-trifluormethyl-1,3-dihydroisobenzofuran;
6-Methoxy-3-trifluormethyl-1,3-dihydrobenzofuran-5-carbaldehyd;
5-Methoxy-1,1-bistrifluormethyl-1,3-dihydroisobenzofuran;
6-Methoxy-3,3-bis(trifluormethyl)-1,3-dihydroisobenzofuran-5-carbaldehyd;
6-Methoxy-1-methyl-1-trifluormethylisochroman;
6-Methoxy-1-methyl-1-trifluormethylisochroman-7-carbaldehyd;
5-Methoxy-1-methyl- -trifluormethyl-1,3-dihydroisobenzofuran;
6-Methoxy-3-methyl-3 -trifluormethyl-1,3-dihydroisobenzofuran-5-carbaldehyd;
1-Trifluormethyl-5-methoxy-1-phenyl-1,3-dihydroisobenzofuran;
3-Trifluormethyl-6-methoxy-3-phenyl-1,3-dihydroisobenzofuran-5-carbaldehyd;
5-Acetyl-3-methyl-6-methoxy-3-trifluormethyl-1,3-dihydroisobenzofuran;
(1R)-6-Methoxy-1-methyl-1-trifluormethylisochroman;
(1R)-6-Methoxy-1-methyl-1-trifluormethylisochroman-7-carbaldehyd;
(1S)-6-Methoxy-1-methyl-1-trifluormethylisochroman;
(1S)-6-Methoxy-1-methyl-1-trifluormethylisochroman-7-carbaldehyd;
(1R)-5-Methoxy-1-methyl-1-trifluormethyl-1,3-dihydroisobenzofuran;
(1R)-6-Methoxy-3-methyl-3-trifluormethyl-1,3-dihydroisobenzofuran-5-carbaldehyd;
(1S)-5-Methoxy-1-methyl-1-trifluormethyl-1,3-dihydroisobenzofuran; und
(1S)-6-Methoxy-3-methyl-3-trifluormethyl-1,3-dihydroisobenzofuran-5-carbaldehyd.

8. Arzneimittel, umfassend eine Verbindung der Formel (I), wie in Anspruch 1 definiert, oder ein pharmazeutisch verträgliches Salz davon und einen pharmazeutisch verträglichen Träger.

9. Mittel gemäß Anspruch 8, das geeignet ist zur Behandlung einer Störung oder eines Zustandes in einem Säugetier, für die (den) ein Substanz P-Antagonist indiziert ist.

10. Mittel gemäß Anspruch 9, wobei die Störung oder der Zustand ausgewählt ist aus Herzkreislauferkrankungen, allergischen Störungen, Angiogenesis, gastrointestinalen Störungen, Störungen des Zentralnervensystems, Entzündungserkrankungen, Erbrechen bzw. Übelkeit, Harninkontinenz, Schmerzen, Migräne, schweren Angstzuständen, stressbedingten Störungen, Angstzuständen, Major Depressionen, Major Depressionen mit Angstzuständen, Depression, Sonnenbrand, sexueller Funktionsstörung, bipolaren Störungen, Störungen auf Grund von Arzneimitteiund/oder Drogengebrauchs bzw. -missbrauchs, schizophrenen Störungen, Bewegungsstörungen, kognitiven Störungen und Erkrankungen, Störungen und nachteiligen Zuständen, die durch Heliobacter pylori hervorgerufen wurden.

11. Verbindung der Formel (I), wie in Anspruch 1 definiert, zur Verwendung als Medikament.

12. Verwendung einer Verbindung der Formel (I), wie in Anspruch 1 definiert, zur Herstellung eines Medikaments zur Behandlung einer Störung oder eines Zustandes in einem Säugetier, für die (den) ein Substanz P-Antagonist indiziert ist.

13. Verwendung nach Anspruch 12. wobei die Störung oder Zustand ausgewählt sind aus Herzkreislauferkrankungen, allergischen Störungen, Angiogenesis, gastrointestinalen Störungen, Störungen des Zentralnervensystems, Entzündungserkrankungen, Erbrechen bzw. Übelkeit, Harninkontinenz, Schmerzen, Migräne, schweren Angstzuständen, stressbedingten Störungen, Angstzuständen, Major Depressionen, Major Depressionen mit Angstzuständen, Depression, Sonnenbrand, sexueller Funktionsstörung, bipolaren Störungen, Störungen auf Grund von Arzneimittel- und/oder Drogengebrauchs bzw. missbrauchs, schizophrenen Störungen, Bewegungsstörungen, kognitiven Störungen und Erkrankungen, Störungen und nachteiligen Zuständen, die durch Heliobacter pylori hervorgerufen wurden.

## Revendications

1. Composé de formule (I) 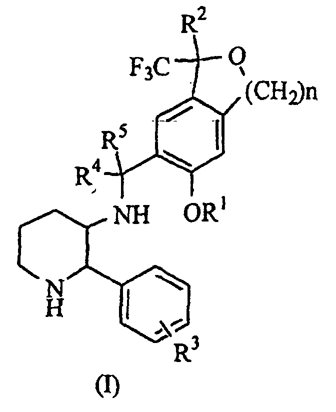 et ses sels pharmaceutiquement acceptables, formule dans laquelle
R¹ représente un groupe alkyle en C₁ à C₆ ;
R² représente l'hydrogène, un groupe alkyle en C₁ à C₆, halogénalkyle en C₁ à C₆ ou phényle ;
R³ représente l'hydrogène ou un groupe halogéno ;
R⁴ et R⁵ représentent indépendamment l'hydrogène, un groupe alkyle en C₁ à C₆ ou halogénalkyle en Ci à C₆ ; et
n est égal à 1, 2 ou 3.

2. Composé suivant la revendication 1, dans lequel
R¹ représente un groupe alkyle en C₁ à C₃ ;
R² représente l'hydrogène, un groupe alkyle en C₁ à C₃, halogénalkyle en C₁ à C₃ ou phényle ;
R³ représente l'hydrogène ou le fluor ;
R⁴ et R⁵ représentent indépendamment l'hydrogène, un groupe alkyle en C₁ à C₃ ou halogénalkyle en Ci à C₃ ; et
n est égal à 1 ou 2.

3. Composé suivant la revendication 2, dans lequel R¹ représente un groupe méthyle ; R² représente l'hydrogène, un groupe méthyle, trifluorométhyle ou phényle ; R³ représente l'hydrogène et R⁴ et R⁵ représentent l'hydrogène.

4. Composé suivant la revendication 3, choisi entre :
(2*S*,3*S*)-3-(6-méthoxy-3-trifluorométhyl-1,3-dihydroisobenzofuranne-5-yl)méthylamino-2-phénylpipéridine ou ses sels ;
(2*S*, 3*S*) -3- (6-méthoxy-1-méthyl-1-trifluorométhylisochromane-7-yl)méthylamino-2-phénylpipéridine ou ses sels ;
(2*S*, 3*S*)-3-(6-méthoxy-3-méthyl-3-trifluorométhyl-1,3-dihydro-isobenzofuranne-5-yl)méthylamino-2-phénylpipéridine ou ses sels ;
(2*S*,3*S*)-3-(6-méthoxy-3-phényl-3-trifluorométhyl-1,3-dihydro-isobenzofuranne-5-yl)méthylamino-2-phénylpipéridine ou ses sels ; et
(2*S*,3*S*)-3-[1-(6-méthoxy-3-méthyl-3-trifluorométhyl-1,3-dihydro-isobenzofuranne-5-yl)éthylamino]-2-phénylpipéridine ou ses sels.

5. Composé suivant la revendication 4, qui est
la (2*S*,3*S*) -3- [(1R)-6-méthoxy-1-méthyl-1-trifluorométhylisochromane-7-yl]méthylamino-2-phénylpipéridine ou ses sels ; ou
la (2*S*,3*S*)-3-[(3R)-(6-méthoxy-3-méthyl-3-trifluorométhyl-1,3-dihydro-isobenzofuranne-5-yl)]méthylamino-2-phénylpipéridine ou ses sels.

6. Composé de formule (III) : 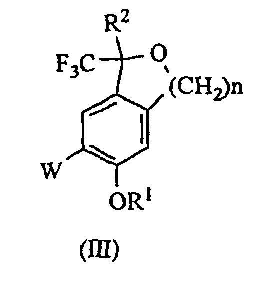 dans laquelle W représente l'hydrogène ou un groupe Q(O=)C- dans lequel Q représente H, un groupe alkyle en C₁ à C₆ ou halogénalkyle en C₁ à C₆ ; R¹ représente un groupe alkyle en C₁ à Ce ; R² représente l'hydrogène, un groupe alkyle en C₁ à C₆, halogénalkyle en C₁ à C₆ ou phényle ; et n est égal à 1, 2 ou 3.

7. Composé suivant la revendication 6, qui est choisi entre les suivants :
5-méthoxy-1-trifluorométhyl-1,3-dihydroisobenzofuranne ;
6-méthoxy-3-trifluorométhyl-1,3-dihydrobenzofuranne-5-carbaldéhyde ;
5-méthoxy-1,1-bis-trifluorométhyl-1,3-dihydroisobenzofuranne ;
6-méthoxy-3,3-bis(trifluorométhyl)-1,3-dihydroisobenzofuranne-5-carbaldéhyde ;
6-méthoxy-1-méthyl-1-trifluorométhylisochromane ;
6-méthoxy-1-méthyl-1-trifluorométhylisochromane-7-carbaldéhyde ;
5-méthoxy-1-méthyl-1-trifluorométhyl-1,3-dihydro-isobenzofuranne ;
6-méthoxy-3-méthyl-3-trifluorométhyl-1,3-dihydro-isobenzofuranne-5-carbaldéhyde ;
1-trifluorométhyl-5-méthoxy-1-phényl-1,3-dihydro-isobenzofuranne ;
3-trifluorométhyl-6-méthoxy-3-phényl-1,3-dihydro-isobenzofuranne-5-carbaldéhyde ;
5-acétyl-3-méthyl-6-méthoxy-3-trifluorométhyl-1,3-dihydro-isobenzofuranne ;
(1R)-6-méthoxy-1-méthyl-1-trifluorométhylisochromane ;
(1R)-6-méthoxy-1-méthyl-1-trifluorométhylisochromane-7-carbaldéhyde ;
(1S) -6-méthoxy-1-méthyl-1-trifluorométhylisochromane ;
(1S)-6-méthoxy-1-méthyl-1-trifluorométhylisochromane-7-carbaldéhyde ;
(1R)-5-méthoxy-1-méthyl-1-trifluorométhyl-1,3-dihydro-isobenzofuranne ;
(1R)-6-méthoxy-3-méthyl-3-trifluorométhyl-1,3-dihydro-isobenzofuranne-5-carbaldéhyde ;
(1S)-5-méthoxy-1-méthyl-1-trifluorométhyl-1,3-dihydro-isobenzofuranne ; et
(1S)-6-méthoxy-3-méthyl-3-trifluorométhyl-1,3-dihydro-isobenzofuranne-5-carbaldéhyde.

8. Composition pharmaceutique comprenant un composé de formule (I) répondant à la définition suivant la revendication 1, ou un de ses sels pharmaceutiquement acceptables, et un support pharmaceutiquement acceptable.

9. Composition suivant la revendication 8, apte au traitement, chez un mammifère, d'un trouble ou d'une affection pour lequel un antagoniste de la substance P est indiqué.

10. Composition suivant la revendication 9, dans laquelle le trouble ou l'affection est choisi entre des maladies cardiovasculaires, des troubles allergiques, l'angiogénèse, des troubles gastro-intestinaux, des troubles du système nerveux central, des maladies inflammatoires, les vomissements, l'incontinence urinaire, la douleur, la migraine ; des troubles anxieux graves, des troubles dus à un stress, l'anxiété, des troubles dépressifs majeurs, des troubles dépressifs majeurs accompagnés d'anxiété, la dépression, un érythème solaire, un dysfonctionnement sexuel, des troubles bipolaires, la dépendance de substances, des troubles schizophréniques, des troubles du mouvement, des troubles cognitifs et des maladies, affections et états néfastes provoqués par *Helicobacter pylori.*

11. Composé de formule (I) répondant à la définition suivant la revendication 1, destiné à être utilisé comme médicament.

12. Utilisation d'un composé de formule (I) répondant à la définition suivant la revendication 1 pour la la préparation d'un médicament destiné au traitement, chez un mammifère, d'un trouble ou d'une affection pour lequel un antagoniste de la substance P est indiqué.

13. Utilisation suivant la revendication 12, dans laquelle le trouble ou l'affection est choisi entre des maladies cardiovasculaires, des troubles allergiques, l'angiogénèse, des troubles gastro-intestinaux, des troubles du système nerveux central, des maladies inflammatoires, les vomissements, l'incontinence urinaire, la douleur, la migraine, des troubles anxieux graves, des troubles dus à un stress, l'anxiété, des troubles dépressifs majeurs, des troubles dépressifs majeurs accompagnés d'anxiété, la dépression, un érythème solaire, un dysfonctionnement sexuel, des troubles bipolaires, la dépendance de substances, des troubles schizophréniques, des troubles du mouvement, des troubles cognitifs et des maladies, affections et états néfastes provoqués par *Helicobacter pylori.*
